# EUROPEAN PATENT APPLICATION

(11) **EP 4 365 596 A1**
(43) Date of publication of application: **08.05.2024**
(21) Application number: 22833276.3
(22) Date of filing: 30.06.2022
(51) Int. Cl.: G01N 33/68, A61K 45/00, A61P 13/00, A61P 13/12, A61P 37/06, C07K 16/18, G01N 33/53

(54) **BIOMARKER FOR DETECTING TUBULOINTERSTITIAL DISORDER AND USE THEREOF**

(30) Priority: 30.06.2021 JP 2021108341
(71) Applicant: PHC Corporation, Toon-shi Ehime 791-0395 (JP)
(72) Inventor: TANAKA Akihito, Nagoya-shi, Aichi 464-8601 (JP); FURUHASHI Kazuhiro, Nagoya-shi, Aichi 464-8601 (JP); MARUYAMA Shoichi, Nagoya-shi, Aichi 464-8601 (JP); SAWA Yuriko, Nagoya-shi, Aichi 464-8601 (JP); SHIRAKAWA Kamon, Toon-shi, Ehime 791-0395 (JP)
(74) Representative: Gille Hrabal Partnerschaftsgesellschaft mbB Patentanwälte
(86) International application number: PCT/JP2022/026214
(87) International publication number: WO 2023/277130

(57) **Abstract**

Provided are a novel biomarker that can conveniently detect a tubulointerstitial disorder or that can grasp the disease state of a kidney disease using a urine sample, and a use thereof. The biomarker is sCD14-ST in urine.

## Description

### TECHNICAL FIELD

The present invention relates to a biomarker for detecting tubulointerstitial disorders and a use thereof.

### BACKGROUND ART

The functions of the kidneys are largely divided into those of water regulation and waste discharge, and hormone production. Urinary waste discharge also has functions of maintaining the electrolytes in the body fluid at an appropriate concentration and keeping the body in a mildly alkaline state. As hormones, the kidneys produce erythropoietin that makes blood, and is also responsible for the activation of vitamin D. Therefore, when such renal functions decline, hypertension, edema, proteinuria, hematuria, anemia, or hypocalcemia occur. Chronic kidney disease (hereinafter also referred to as "CKD") is a state in which renal functions gradually decline, and the process is common regardless of the cause. The disease progresses gradually from the initial stage of renal failure to renal disorder, in which the kidneys eventually stop functioning and renal replacement therapy (kidney transplantation, dialysis) is required. Renal function is diagnosed as CKD when, based on the estimated glomerular filtration rate (eGFR; calculated from Cr concentration, age, and gender) calculated from serum creatinine (Cr) concentration as well as urinary protein, hematuria, and renal imaging diagnosis, eGFR is 60 mL/min/1.73 m² or less or abnormalities continue for 3 months or more. It is estimated that there are approximately 13 million CKD patients in Japan, and approximately 350,000 patients are undergoing dialysis nationwide. Renal function declines with aging, and there is currently no treatment that can directly restore renal function. Chronic glomerulonephritis used to be the most common cause of decreased renal function, but now diabetic nephropathy, and nephrosclerosis caused by hypertension are increasing due to changes in lifestyle. However, since there is no treatment to restore renal function, the key points of treatment for CKD are (1) prevention of progression of renal failure, (2) reduction of cardiovascular disease risk, (3) treatment of symptoms (treatment of renal failure symptoms and complications), and (4) preparation for future renal replacement therapy (transplantation, dialysis). Among them, prevention of end-stage renal failure is the goal of CKD treatment. Therefore, lifestyle improvement and dietary therapy are used in the treatment of CKD. It is also important to treat hypertension and diabetes, which aggravate renal function. Patients with CKD should ideally consume less than 6 g of salt per day. Care should also be taken not to consume too much protein and potassium. In addition, attention to dehydration and energy intake is necessary, and it is important to maintain compliance in daily life.

The best way to grasp the cause and progression of CKD is a definitive diagnosis by renal biopsy. However, the patients who can undergo renal biopsy are limited by kidney conditions, the risk of bleeding, or the like, and the 4-5 day hospitalization, 40,000-70,000 yen co-payment (30% of the total cost), the risk of bleeding after renal biopsy, and the like place a heavy burden on patients. Therefore, less than half of the patients for whom the definitive diagnosis is recommended by renal biopsy can actually be performed. It has been reported that only approximately 5% of patients on dialysis have been definitively diagnosed by renal biopsy. To avoid dialysis, it is important to maintain renal function and delay the decline of renal function, and it is necessary to take measures at an early stage. Although it is desirable to perform renal biopsy to diagnose the etiology and determine a treatment plan, it is not easy to do so. Currently used diagnostic methods use proteinuria (albuminuria), hematuria, renal biopsy, serum creatinine (Cr) value, eGFR value, and the like as indicators. Indicators other than imaging diagnosis by renal biopsy are markers reflecting the overall picture of renal failure, and they do not have diagnostic sensitivity enough to make a sensitive diagnosis of the location and degree of damage (especially, damage to the tubulointerstitium) in patients with kidney disease whose renal function needs to be closely examined, and the diagnosis is made comprehensively based on laboratory and clinical findings. As a result of the delay in performing renal biopsy, or in patients with risk factors that preclude renal biopsy, it may take too long to initiate effective treatment and the treatment outcome may be poor.

Despite rapid progress in diagnosis and treatment, approximately 35,000 new patients are forced to undergo dialysis treatment each year, and the challenge of how to reduce the number of patients who are shifted to dialysis is an issue that must be overcome both for the patients and for the health care economy. Many of diseases that present with CKD are lifestyle-related diseases such as hypertension, diabetes, and the like, which require lifestyle management. The conditions must be monitored on a monthly basis after the onset of the disease, and there is currently no way to prevent dialysis without early diagnosis and early therapeutic intervention of CKD. To cope with this, the need to classify the severity of CKD into stages, to grasp the disease conditions at an earlier stage, and to intervene earlier in treatment has been especially emphasized in recent years.

Currently, the diagnosis of CKD is usually made by eGFR, which is calculated based on elevated serum Cr as an indicator. However, eGFR only indicates the status of renal function and is not an indicator of the cause of CKD, and thus there is a lack of information for the selection of a treatment method. Therefore, eGFR is not a sufficient diagnostic marker for CKD, and there is an urgent need to establish a biomarker that can evaluate the cause of dysfunction. The complex patient background and the diversity of etiology of CKD, as well as the lack of biomarkers that can diagnose the conditions of CKD at an early stage, make it impossible to intervene at an early stage.

On the other hand, acute kidney injury (hereinafter also referred to as "AKI") is a syndrome with rapid loss of renal function and tissue damage within a few hours to a few days. Based on the cause of renal dysfunction, AKI is classified into three types: "prerenal renal failure", in which blood flow to the kidneys is reduced; "renal renal failure", in which the kidneys themselves become abnormal; and "postrenal renal failure", in which the kidneys are damaged due to blockage of the channels through which urine produced by the kidneys flows (urinary tract: renal pelvis, ureters, bladder, and urethra). Symptoms include decreased urine volume, swelling (edema), decreased appetite, general malaise, and the like, and blood tests show high values of blood urea nitrogen (BUN), serum creatinine (Cr), and potassium (K). The first cause is decreased blood flow to the kidneys due to dehydration or bleeding (prerenal), the second cause is decreased renal function due to inflammation of the kidneys, tubular cell damage, or the like (renal), and the third cause is due to obstruction of the urinary tract system (postrenal). In many cases of the pre-renal and post-renal, renal function can be restored by clarifying the cause and by taking appropriate coping therapy. On the other hand, in severe cases of renal dysfunction, hemodialysis is usually performed because of the need to remove waste products and excess water from the body. Acute kidney injury can often be expected to recover to the original renal function with appropriate treatments, but acute kidney injury in intensive care units or the like, such as kidney injury or the like associated with sepsis, generally has a poor prognosis. It is necessary to identify the cause as soon as possible and to select a treatment for it, but the type and extent of tissue damage cannot be grasped because renal biopsy is difficult to perform due to rapid changes in symptoms, bleeding tendency, or the like. Therefore, treatment options are limited, for example, the dialysis treatment or the like has to be chosen as emergency measures. In addition, it has recently been recognized that AKI is not reversible disease conditions but a risk factor for the development and progression of CKD in the long term, and that there are issues to be resolved to improve the prognosis of AKI.

Recently, the usefulness of neutrophil gelatinase-associated lipocaline (NGAL; Non-patent literature 1), liver type fatty acid-binding protein (L-FABP; Non-patent literature 2), and the like as urinary biomarkers that can diagnose AKI or CKD has been reported. However, it is not sufficient to grasp renal damage and the severity for appropriate and timely intervention such as treatment of the like, which is required in clinical practice, and a new diagnostic biomarker for kidney disease and a biomarker that can evaluate the cause of kidney disease have been sought.

By the way, it has been disclosed that sCD14-ST (hereinafter also referred to as "presepsin (registered trademark)") in samples is measured to diagnose sepsis (Non-patent literature 1 and Patent literature 1). It has also been disclosed that sCD14-ST is measured using its specific antibodies (Patent literature 1 and Patent literature 2). Furthermore, it has been reported from a dog study that presepsin in blood is excreted into urine by renal metabolism as a physiological mechanism (Patent literature 3), and it has also been reported that the blood presepsin value increases with increased renal failure (GFR stage classification) and shows negative correlation with the eGFR value (Non-patent literature 2). It is generally believed that the excretion of substances of renal metabolism is restricted by renal failure, and that the amount of excretion decreases as urine output decreases, resulting in an increase in blood concentrations, and it was presumed that blood presepsin also increases with a decrease in eGFR. In fact, it has been reported that blood creatinine can compensate the effect of renal failure by compensating the presepsin value, and does not affect the diagnosis of sepsis (Non-patent literature 3).

### CITATION LIST

### PATENT LITERATURE

[Patent literature 1] Japanese Patent No. 4040666
[Patent literature 2] JP 2005-106694 A
[Patent literature 3] WO 2012157751

### NON-PATENT LITERATURE

[Non-patent literature 1] J Infect Chemother 2005; 11: 234-238.
[Non-patent literature 2] PLoS One.2015; 10(6): e0129159.
[Non-patent literature 3] The Journal of Medical Investigation 2021; 68(1.2): 105-111

### SUMMARY OF INVENTION

### TECHNICAL PROBLEM

Against the above background, an object of the present invention is to provide a novel biomarker that can conveniently detect tubulointerstitial disorders using urine samples, and its application. Furthermore, another object of the present invention is to provide a novel biomarker capable of conveniently grasping the conditions of kidney disease using urine specimens, and its application.

### SOLUTION TO PROBLEM

In view of the above objects, despite the previous findings, the inventors focused on presepsin in blood, and presepsin in urine (hereinafter also referred to as uP-SEP), and intensively studied its potential as a biomarker. As a result, as described in the Examples below, urinary presepsin was found to be clearly related to the degree of renal damage, and changed sensitively reflecting the degree of damage. Furthermore, CKD patients showed a clear negative correlation with the eGFR value even after creatinine compensation, reflecting the severity of renal failure, whereas blood presepsin showed no relationship with the eGFR value after creatinine compensation. It was a surprising finding that blood presepsin did not reflect the degree of renal damage, and only urinary presepsin significantly correlated with the degree of renal damage.

As reported in Non-patent literature 3, it has conventionally been believed that presepsin is produced at the site of infection, and that the blood presepsin concentration is affected by urine volume or the like, but has no direct relationship with kidney disease. Furthermore, since urinary presepsin was presumed to be a metabolite of blood presepsin, changes in urinary presepsin values were thought to reflect blood presepsin values. From the results of the inventors' study, it was a surprising finding that based on the urinary presepsin value, renal damage can be detected and the conditions of kidney disease can be grasped, and furthermore, tubulointerstitial disorders can be specifically detected, which could not be inferred from known findings.

Furthermore, the present inventors investigated the possibility of urinary presepsin as a biomarker, and found that the urinary presepsin concentrations were elevated in patients with various kidney diseases who underwent renal biopsy than those in normal subjects, and that the highest urinary presepsin concentrations were observed particularly in interstitial nephritis patients with tubulointerstitial disorders. Furthermore, the urinary presepsin concentration increased according to kidney diseases reported to have a high degree of tubulointerstitial disorders (kidney disease derived from ANCA-associated vasculitis or the like), and the urinary presepsin concentration was lowest in IgA nephropathy in which the degree of tubulointerstitial disorders was presumed to be relatively low. In other words, it was shown that the degree of tubulointerstitial disorders in various kidney diseases can be grasped based on the urinary presepsin concentration. Similar results were also obtained with the urinary presepsin/creatinine (uP-SEP/Cr) value compensated by urinary creatinine to eliminate the influence of urine volume on the urinary presepsin value, indicating that it is a specific marker for tubulointerstitial disorders.

Furthermore, the sensitivity of uP-SEP/Cr in distinguishing tubulointerstitial nephritis (also referred to as interstitial nephritis) was 85.7%, the specificity was 66.2%, and the AUC of ROC curve was 0.826, indicating that urinary presepsin is an unprecedented and highly useful single biomarker to detect interstitial nephritis.

Furthermore, based on the findings of renal damage in renal biopsy, urinary presepsin was able to distinguish between groups with and without interstitial damage (including with glomerular damage) with significant difference, indicating that urinary presepsin is an unprecedented and highly useful single biomarker to detect tubulointerstitial disorders.

Furthermore, the degree of increase in uP-SEP/Cr values between groups in the degree of damage of interstitial nephritis showed that the increase was more rapid in inflammatory cell infiltration, indicating that monitoring the uP-SEP/Cr value is useful for grasping the degree of progress of tubulointerstitial disorders in the acute stage, and can be used to judge the initiation of treatment and to judge the effectiveness of treatment.

Furthermore, the present inventors investigated the relationship between the degree of tubular atrophy or the degree of inflammatory cell infiltration obtained from renal biopsy and the urinary presepsin concentration, and found a surprising finding that the urinary presepsin concentration increased in patients with high degree of atrophy or infiltration. In this way, it was shown that tubulointerstitial disorders can be conveniently grasped at an early stage without performing histological diagnosis of renal biopsy.

Furthermore, the uP-SEP/Cr value can detect tubulointerstitial disorders in patients with IgA nephropathy, indicating that the uP-SEP/Cr value is useful for the diagnosis of severe IgA nephropathy.

Furthermore, in CKD patients with diabetic nephropathy, nephrosclerosis, or the like without renal biopsy, the relationship between patients' uP-SEP/Cr values and subsequent renal function progression was analyzed, and the rate of decline in eGFR indicated that patients with higher uP-SEP/Cr values had higher degrees of renal dysfunction, indicating that the uP-SEP/Cr value is related to the degree of damage of renal function in patients and can reflect prognosis.

As described above, as a result of the inventors' intensive study, it is clear that urinary presepsin is a useful indicator (biomarker) for grasping tubulointerstitial disorders.

On the other hand, the results of an experiment examining the relationship between the rate of change in eGFR and uP-SEP/Cr values in patients with chronic kidney disease (CKD) showed that the uP-SEP/Cr value is effective as an indicator for grasping the severity of CKD.

Furthermore, the temporal observation of the changes in eGFR and uP-SEP/Cr values in CKD patients showed that a decreasing trend of eGFR was observed in a group of patients with an increasing trend of the uP-SEP/Cr value, while an increasing trend of eGFR was observed in a group of patients with a decreasing trend of the uP-SEP/Cr values, indicating that the change in uP-SEP/Cr value can be used to grasp the severity of CKD patients and to monitor their conditions. Furthermore, in CKD patients diagnosed with drug-induced renal disorder, the uP-SEP/Cr values decreased and eGFR increased after drug discontinuation, indicating that the severity of CKD patients can be grasped and that treatment options such as drug modification, discontinuation, and the like can be selected by observing the change in uP-SEP/Cr value.

From this result, the uP-SEP/Cr value can detect tubulointerstitial disorders, indicating that the uP-SEP/Cr value is useful as a marker for tubulointerstitial disorders in CKD patients and can be used for monitoring patients.

The uP-SEP/Cr value can detect tubulointerstitial disorders, indicating that the uP-SEP/Cr value is useful as a marker for tubulointerstitial disorders in AKI patients and can be used to grasp the conditions of patients.

As described above, as a result of the inventors' intensive study, it was found that urinary presepsin is a very good biomarker for the detection of tubulointerstitial disorders.

Based on the above findings of the inventors, the present invention has been completed.

This invention relates to the following:
[1] A method for detecting a tubulointerstitial disorder by measuring a concentration of sCD14-ST in urine of a subject (preferably a value obtained by dividing the concentration of sCD14-ST in urine by a urinary creatinine value).
[2] The method of [1], wherein the tubulointerstitial disorder is a chronic tubulointerstitial disorder.
[3] The method of [1] or [2], wherein the subject is a patient suspected of having a kidney disease or a patient with a kidney disease.
[4] The method of [1] or [2], wherein the subject is a patient suspected of having one or more kidney diseases selected from the group consisting of nephrosclerosis, IgA nephropathy, diabetic nephropathy, membranous nephropathy, ANCA-associated vasculitis, focal segmental glomerulosclerosis, minimal change nephrotic syndrome, lupus nephritis, tubulointerstitial nephritis, Sjogren's syndrome, vascular renal disease, idiopathic TINU syndrome, and IgG4-related nephritis, or a patient with said kidney diseases.
[5] The method of [1], wherein the subject is a patient suspected of having a kidney disease that is an acute tubulointerstitial nephritis or a chronic tubulointerstitial nephritis, or a patient with said kidney disease.
[6] The method of [1] or [5], wherein the subject is a patient suspected of having a kidney disease that is an acute drug-induced tubulointerstitial nephritis or a chronic drug-induced tubulointerstitial nephritis, or a patient with said kidney disease.
[7] A method for monitoring a tubulointerstitial disorder by measuring a concentration of sCD14-ST in urine of a subject (preferably a value obtained by dividing the concentration of sCD14-ST in urine by a urinary creatinine value).
[8] A method for selecting a treatment for a tubulointerstitial disorder by measuring a concentration of sCD14-ST in urine of a subject (preferably a value obtained by dividing the concentration of sCD14-ST in urine by a urinary creatinine value).
[9] A method for grasping severity of a kidney disease by measuring a concentration of sCD14-ST in urine of a subject (preferably a value obtained by dividing the concentration of sCD14-ST in urine by a urinary creatinine value).
[10] A method for detecting a kidney disease by measuring a concentration of sCD14-ST in urine of a subject (preferably a value obtained by dividing the concentration of sCD14-ST in urine by a urinary creatinine value).
[11] A method for detecting a kidney disease by measuring a concentration of sCD14-ST in urine of a subject (preferably a value obtained by dividing the concentration of sCD14-ST in urine by a urinary creatinine value), wherein the kidney disease is one or more kidney diseases selected from the group consisting of nephrosclerosis, IgA nephropathy, diabetic nephropathy, membranous nephropathy, ANCA-associated vasculitis, focal segmental glomerulosclerosis, minimal change nephrotic syndrome, lupus nephritis, tubulointerstitial nephritis, Sjogren's syndrome, vascular renal disease, idiopathic TINU syndrome, and IgG4-related nephritis.
[12] A method for detecting tubulointerstitial nephritis
   by measuring a concentration of sCD14-ST in urine of a subject (preferably a value obtained by dividing the concentration of sCD14-ST in urine by a urinary creatinine value).
[13] A method for detecting a tubulointerstitial disorder comprising:
   measuring a concentration of sCD14-ST in urine collected from a patient suspected of having a tubulointerstitial disorder or a patient with a tubulointerstitial disorder (preferably a value obtained by dividing the concentration of sCD14-ST in urine by a urinary creatinine value), and
   judging presence of a tubulointerstitial disorder when the concentration of sCD14-ST (preferably the value obtained by dividing the concentration of sCD14-ST in urine by a urinary creatinine value) is higher compared to healthy subjects.
[14] A method for detecting and treating a tubulointerstitial disorder comprising:
   measuring a concentration of sCD14-ST in urine collected from a patient suspected of having a tubulointerstitial disorder or a patient with a tubulointerstitial disorder (preferably a value obtained by dividing the concentration of sCD14-ST in urine by a urinary creatinine value),
   judging presence of a tubulointerstitial disorder when the concentration of sCD14-ST (preferably the value obtained by dividing the concentration of sCD14-ST in urine by a urinary creatinine value) is higher compared to healthy subjects, and
   performing immunosuppressive therapy when the presence of the tubulointerstitial disorder is judged.
[15] A method for detecting a kidney disease comprising:
   measuring a concentration of sCD14-ST in urine collected from a patient suspected of having a kidney disease or a patient with a kidney disease (preferably a value obtained by dividing the concentration of sCD14-ST in urine by a urinary creatinine value), and
   judging presence of a kidney disease when the concentration of sCD14-ST (preferably the value obtained by dividing the concentration of sCD14-ST in urine by a urinary creatinine value) is higher compared to healthy subjects.
[16] A method for detecting and treating a kidney disease comprising:
   measuring a concentration of sCD14-ST in urine collected from a patient suspected of having a kidney disease or a patient with a kidney disease (preferably a value obtained by dividing the concentration of sCD14-ST in urine by a urinary creatinine value),
   judging presence of a kidney disease when the concentration of sCD14-ST (preferably the value obtained by dividing the concentration of sCD14-ST in urine by a urinary creatinine value) is higher compared to healthy subjects, and
   performing immunosuppressive therapy when the presence of the kidney disease is judged.
[17] The method of any one of [1] to [16], wherein the concentration of sCD14-ST (preferably the value obtained by dividing the concentration of sCD14-ST in urine by a urinary creatinine value) is measured by an immunoassay.
[18] Use of a concentration of sCD14-ST in urine (preferably a value obtained by dividing the concentration of sCD14-ST in urine by a urinary creatinine value) as a marker for detecting a tubulointerstitial disorder.
[19] A kit for detecting a tubulointerstitial disorder, comprising:
   (a) an antibody specific for sCD14-ST,
   (b) standard data correlating a concentration of sCD14-ST in urine (preferably a value obtained by dividing the concentration of sCD14-ST in urine by a urinary creatinine value) with a degree of renal damage, and
   (c) an instruction manual.
[20] A kit for detecting a tubulointerstitial disorder, comprising:
   (a) an antibody specific for sCD14-ST,
   (b) standard data correlating a concentration of sCD14-ST in urine (preferably a value obtained by dividing the concentration of sCD14-ST in urine by a urinary creatinine value) with severity of a kidney disease, and
   (c) an instruction manual.

The term "human sCD14-ST" (also referred to as presepsin (registered trademark)) as used herein means the "soluble CD14 antigen of the first embodiment" described in Japanese Patent No. 4040666, and more specifically, it is the soluble CD14 antigen having the following features 1) to 3):
1) having a molecular weight of 13±2 kDa by SDS-PAGE under non-reducing conditions,
2) having the amino acid sequence of SEQ ID NO: 1 in the N-terminal sequence, and
3) binding specifically to an antibody prepared using a peptide consisting of 16 amino acid residues of SEQ ID NO: 2 as an antigen.

In connection with this, "sCD14-ST" means human sCD14-ST unless otherwise specified.

SEQ ID NO: 1: 
Thr Thr Pro Glu Pro Cys Glu Leu Asp Asp Glu
1               5                   10
SEQ ID NO: 2:

Arg Val Asp Ala Asp Ala Asp Pro Arg Gln Tyr Ala Asp Thr Val
1               5                   10                  15
Lys

The term "tubulointerstitial disorders" as used herein means tubulointerstitial disorders in which the causes of kidney disease conditions can be evaluated, for example, tubulointerstitial disorders with inflammatory cell infiltration into the interstitium and tubular atrophy, based on findings of damage evaluated by renal biopsy. The degree of tubulointerstitial disorders can be evaluated by histological evaluation of the degree of inflammatory cell infiltration into the interstitium and tubular atrophy, by Banff classification-like scoring, or by tubular atrophy and interstitial fibrosis (T-score) in the Oxford classification. Tubulointerstitial disorders include acute or chronic tubulointerstitial disorders, drug-induced or non-drug-induced tubulointerstitial disorders, and the like.

The term "detection of tubulointerstitial disorders" as used herein is not limited as long as it is to grasp tubulointerstitial disorders in order to evaluate the causes of kidney disease conditions, and includes, for example, to detect the presence or absence of tubulointerstitial disorders, to detect the degree of tubulointerstitial disorders, and the like. The term "detection of tubulointerstitial disorders" as used herein does not exclude the presence of other renal disorders when detecting tubulointerstitial disorders. Depending on the type and severity of kidney disease, tubulointerstitial disorders and other renal disorders may occur together.

The term "the degree of renal damage" as used herein means the degree of renal damage from which the causes of kidney disease conditions can be evaluated, and the classification is based on the findings of damage evaluated by renal biopsy according to the guidelines provided by the Japanese Society of Nephrology, various academic literatures, or the like. For example, the degree of damage in tubulointerstitial disorders is classified according to the degree of inflammatory cell infiltration into the interstitium, tubular atrophy, or the like.

The term "severity" as used herein is an indicator to evaluate the conditions and stage of kidney disease as classified by the Clinical Practice Guidelines for CKD or the Clinical Practice Guidelines for AKI provided by the Japanese Society of Nephrology or the like. It can also be used to select a management method or a treatment method for kidney disease.

The term "kidney disease" as used herein is a disease in which the function of the kidneys is impaired due to damage to the glomeruli and renal tubules of the kidneys. Kidney disease includes "acute renal failure" in which symptoms occur rapidly and worsen by the hour or day, and "chronic renal failure" in which the disease progresses slowly and subjective symptoms often do not appear until the disease is near the end stage.

Chronic renal failure is a chronically progressive renal disorder, and has recently been has recently been considered as acute kidney injury (AKI) as chronic kidney disease (CKD). CKD has a wide variety of causes and is closely related to lifestyle-related diseases (diabetes, hypertension, and the like) and metabolic syndrome such as chronic nephritis and the like, and it is known that there are approximately 13 million CKD patients in Japan (one in eight adults aged 20 or older is affected).

The severity of chronic kidney disease (CKD) is recommended to be evaluated by CGA classification based on cause (C), renal function (GFR: G), and proteinuria (albuminuria: A), according to the "Clinical Practice Guidelines 2012" published by the Japanese Society of Nephrology in 2012, or the like. Previously, the stage of chronic kidney disease was evaluated only by renal function classified by eGFR. However, the accuracy and validity of diagnosis have been further improved by evaluating the degree of kidney function in combination with diseases causing kidney disease, such as diabetes, hypertension, or the like, as well as urinary protein status.

The treatment for chronic kidney disease (CKD) is lifestyle improvement, dietary therapy, or drug therapy in the advanced stages (stage 2 to stage 4), and dialysis therapy or kidney transplantation in the end stage (stage 5; renal failure). As the lifestyle improvement, the Guidelines for lifestyle guidance by the Japanese Society of Nephrology provides the following guidelines: avoid excessive exercise in daily life, avoid fatigue, pay attention to infections such as colds or the like, avoid colds, eat luxury foods in moderation, pay attention to food, and the like. The dietary therapy includes adequate caloric intake, protein restriction, salt restriction, potassium and phosphorus restriction, adequate fluid intake, and the like. As the drug therapy, for example, antihypertensive drugs, diuretics, phosphorus adsorbents, potassium adsorbents, erythropoietin preparations, steroids, immunosuppressive agents, Chinese herbal medicines, or the like are used, taking into consideration the conditions, underlying diseases, and the like.

Regardless of the above, the Japanese Society of Nephrology and other organizations have published various clinical practice guidelines for chronic kidney disease, which can be referred to for diagnosis, treatment, prevention, and the like.

Acute renal failure is a rapidly progressive renal disorder, and has recently been recognized as acute kidney injury (AKI). AKI is a broad clinical syndrome defined as "sudden loss of renal function" and classified into three categories according to its pathogenic mechanism: prerenal, renal, and postrenal. Since the treatment strategy for AKI depends on the cause of the disease, it is important to differentiate the disease. Guidelines have been proposed to unify the definition and early diagnosis of AKI. In 2012, Kidney Disease: Improving Global Outcomes (KDIGO) published the Clinical Practice Guidelines for AKI (KDIGO Guidelines), in which the KDIGO criteria were proposed by integrating the previous RIFLE and AKIN criteria. In Japan, the KDIGO criteria are recommended for the diagnosis of AKI in the Clinical Practice Guidelines for AKI 2016 published by the Japanese Society of Nephrology and other organizations.

According to the KDIGO criteria, AKI is defined by increased serum creatinine and decreased urine output, and the severity of AKI is classified into stage 1 to stage 3. It is important to evaluate the appropriate severity of AKI at an early stage and to perform management stratified by disease stage, by measuring serum creatinine and urine output at appropriate times, along with medical history, physical findings, blood and urine tests, and imaging tests (ultrasonography, CT, or the like). It is said that even after improvement of AKI, patients should continue to be evaluated for new onset of CKD or worsening of existing CKD.

In the treatment of AKI, the severity of AKI and the degree of complications should be judged first to determine whether emergency treatment is necessary. When hyperkalemia, metabolic acidosis, severe water overflow, or the like is observed due to AKI, treatment such as drug therapy or the like should be considered immediately regardless of the cause, and dialysis therapy should be performed in some cases. At the same time, the cause of AKI should be identified from medical history, physical findings, and examination findings, and treatment should be initiated immediately. For example, in the case of prerenal acute kidney injury caused by dehydration or hypotension, the patient should be treated with transfusion and, if necessary, hypertensive drugs in parallel with the treatment of its cause. In the case of renal acute renal injury due to inflammation of the kidneys themselves caused by vasculitis (rapidly progressive glomerulonephritis), immunosuppressive therapy such as steroids or the like and plasma exchange therapy to remove antibodies or the like that cause vasculitis are also considered. During the period of recovery from AKI, the patient should be managed with appropriate transfusion and nutritional management, avoiding drugs or the like that interfere with renal function, so as not to adversely affect the kidneys. In the case of postrenal acute kidney injury associated with urinary tract obstruction, the patient should be treated to remove the obstruction to urinary transit.

Regardless of the above, the Japanese Society of Nephrology and other organizations have published various clinical practice guidelines for acute kidney injury, which can be referred to for diagnosis, treatment, prevention, and the like.

### ADVANTAGEOUS EFFECTS OF INVENTION

According to the method of the present invention, tubulointerstitial disorders can be conveniently detected in the early stage of tubulointerstitial disorders with or without renal biopsy. Unlike known biomarkers for detecting renal disorders, urinary presepsin specifically detects tubulointerstitial disorders and can be used as a single biomarker to detect tubulointerstitial nephritis with unprecedented and very high utility.

According to the method of the present invention, with or without renal biopsy, it is possible to detect tubulointerstitial disorders conveniently, to grasp its damage and the degree of the damage, and to select treatment according to the damage.

According to the method of the present invention, with or without renal biopsy, it is possible to monitor tubulointerstitial disorders conveniently, to grasp trends in damage, and to select treatment according to the trends in damage.

According to the method of the present invention, with or without renal biopsy, it is possible to grasp the severity of chronic kidney disease conveniently. Furthermore, it is possible to select treatment according to the severity or trends in severity.

According to the method of the present invention, with or without renal biopsy, it is possible to grasp the conditions of acute renal disorder. Furthermore, it is possible to predict the prognosis based on the conditions or trends in the conditions, and to select treatment.

The biomarker of the present invention can be used for the methods of the invention.

The kit of the present invention can be used for the methods of the invention.

### BRIEF DESCRIPTION OF THE DRAWINGS

[Fig. 1] Figure 1 shows a calibration curve generated by using an automated chemiluminescent immunoassay analyzer (PATHFAST) and prepared by adding a presepsin standard (manufactured by LSI Medience corporation) to a urine sample from a normal subject and sequentially diluting it two-fold with the same urine.
[Fig. 2] Figure 2 is a chromatograph comparing the elution position of presepsin in urine from normal subjects with that of a presepsin standard, which were detected by presepsin ELISA.
[Fig. 3] Figure 3 is a graph comparing eGFR with urinary presepsin (uP-SEP/gCr) or blood presepsin (sP-SEP/Cr) in CKD patients.
[Fig. 4] Figure 4 is a graph comparing urinary P-SEP concentrations (measured values) for various kidney diseases with renal biopsy. AAV: ANCA-associated vasculitis. DMN: diabetic nephropathy. FSGS: focal glomerulosclerosis. IgAN: IgA nephropathy. IN: interstitial nephritis. LN: lupus nephritis. MCNS: minimal change nephrotic syndrome. MN: membranous nephropathy. N: normal subj ects.
[Fig. 5] Figure 5 is a graph showing the result of ROC analysis for determining the cutoff value and its diagnostic performance to differentiate four types of kidney disease in a population of AAV, DMN, lupus nephritis, interstitial nephritis, and normal subjects (measured values).
[Fig. 6] Figure 6 is a graph showing the result of group comparison between various diseases using uP-SEP/Cr.
[Fig. 7] Figure 7 is a graph showing the result of ROC analysis of uP-SEP/Cr in the detection of interstitial nephritis.
[Fig. 8] Figure 8 is a graph showing the result of comparison between with/without findings in renal biopsy and uP-SEP/Cr values.
[Fig. 9] Figure 9 is a graph comparing three groups of patients with renal biopsy: patients with interstitial damage (mild or more), patients without interstitial damage + without glomerular damage, and patients without interstitial damage + with glomerular damage.
[Fig. 10] Figure 10 is a graph comparing the degree of inflammatory cell infiltration or tubular atrophy by renal biopsy with the uP-SEP/Cr values (Vs: minimal).
[Fig. 11] Figure 11 is a graph comparing the degree of inflammatory cell infiltration or tubular atrophy in interstitial disorder with the uP-SEP/Cr values (Vs: minimal).
[Fig. 12] Figure 12 is images of a normal case (left) and an interstitial nephritis case (right) of renal biopsy used for judgement.
[Fig. 13] Figure 13 is a graph comparing the T-score in the Oxford classification with the uP-SEP/Cr values in IgA nephropathy patients (cutoff value: T-score>0).
[Fig. 14] Figure 14 is a graph comparing the treatment progress (eGFR value) with the uP-SEP/Cr values in CDK patients (uP-SEP/Cr values in circles).

### DESCRIPTION OF EMBODIMENTS

Hereinafter, the embodiments of the present invention will be explained in detail, mainly taking the method for detecting tubulointerstitial disorders or the method for detecting kidney disease as examples, but the embodiments of use are not limited to thereto. For example, the present invention includes:
- a method for detecting a tubulointerstitial disorder or a method for detecting a kidney disease, by measuring a concentration of sCD14-ST in urine of a subject (preferably a value obtained by dividing the concentration of sCD14-ST in urine by a urinary creatinine value);
- a method for assisting in a detection of a tubulointerstitial disorder or a method for assisting in a detection of a kidney disease, by measuring a concentration of sCD14-ST in urine of a subject (preferably a value obtained by dividing the concentration of sCD14-ST in urine by a urinary creatinine value);
- a method for measuring a concentration of sCD14-ST in urine of a subject (preferably a value obtained by dividing the concentration of sCD14-ST in urine by a urinary creatinine value) for a detection of a tubulointerstitial disorder or for a detection of a kidney disease;
- an in vitro method for detecting a tubulointerstitial disorder or an in vitro method for detecting a kidney disease, by measuring a concentration of sCD14-ST in urine of a subject (preferably a value obtained by dividing the concentration of sCD14-ST in urine by a urinary creatinine value);
- use of an antibody specific for sCD14-ST in the manufacture of a kit for a detection of a tubulointerstitial disorder or use of an antibody specific for sCD14-ST in the manufacture of a kit for a detection of a kidney disease; and
- a method for measuring a concentration of sCD14-ST in urine of a subject (preferably a value obtained by dividing the concentration of sCD14-ST in urine by a urinary creatinine value) to provide information necessary for a detection of a tubulointerstitial disorder or to provide information necessary for a detection of a kidney disease.

Furthermore, the present invention includes use of an antibody specific for sCD14-ST in the manufacture of a kit for use in the various methods of the present invention.

The various methods of the present invention can be carried out both in vitro and in vivo, but in vitro is preferred.

In the detection method of the present invention (hereinafter referred to as the method of the present invention), presepsin in urine collected from the subject is measured.

The subjects of in the present invention include a patient suspected of having tubulointerstitial disorders or a patient with tubulointerstitial disorders. Specifically, the subjects include a patient suspected of having an acute tubulointerstitial disorder or a patient with an acute tubulointerstitial disorder, a patient suspected of having a chronic tubulointerstitial disorder or a patient with a chronic tubulointerstitial disorder, a patient suspected of having a non-drug-induced tubulointerstitial disorder or a patient with a non-drug-induced tubulointerstitial disorder, a patient suspected of having a drug-induced tubulointerstitial disorder or a patient with a drug-induced tubulointerstitial disorder and the like. For example, drug-induced tubulointerstitial disorders are known to be caused by immunosuppressive drugs such as steroids or the like, aspirin, NSAIDs such as anti-inflammatory analgesics or the like, antibacterial drugs, proton pump inhibitors (PPIs), or the like. They are classified into an acute drug-induced tubulointerstitial disorder, which is acute, and a chronic drug-induced tubulointerstitial disorder, which is either chronic or transitional from acute. The method of the present invention can detect both tubulointerstitial disorders.

The chronic tubulointerstitial disorders often progress without clinical symptoms, and the fact that the method of the present invention can detect tubulointerstitial disorders with or without renal biopsy is a very useful effect.

Chronic kidney diseases with chronic tubulointerstitial disorders include chronic kidney diseases (CKDs), such as
nephrosclerosis, IgA nephropathy, diabetic nephropathy, membranous nephropathy, ANCA-associated vasculitis, focal segmental glomerulosclerosis, minimal change nephrotic syndrome, lupus nephritis, tubulointerstitial nephritis(also referred to as interstitial nephritis), Sjogren's syndrome, vascular renal disease, idiopathic TINU syndrome, IgG4-related nephritis, and the like. In particular, it is preferred because it can distinguish tubulointerstitial nephritis, nephrosclerosis, diabetic nephropathy, membranous nephropathy, ANCA-related vasculitis, minimal change nephrotic syndrome, lupus nephritis, and the like, with severe damage to the tubulointerstitium. In particular, tubulointerstitial nephritis is highly desirable because it can be differentiated only by the concentration of urinary presepsin. The above-mentioned "concentration of urinary presepsin" includes not only the urinary presepsin concentration before compensation by urinary creatinine but also, for example, the urinary presepsin concentration/urinary creatinine (uP-SEP/Cr) value which is the value compensated by urinary creatinine, and the like.

Kidney diseases with acute tubulointerstitial disorders include acute kidney injury (AKI). In particular, in the prerenal or renal stage, conditions with damage to the renal tubules or interstitium are preferred.

The method of the present invention is preferred, because it can detect tubulointerstitial disorders regardless of the type of kidney disease, and the type and degree of the damage can be grasped. Furthermore, the method is also preferred, because it is possible to select a treatment method based on the grasp of the type and degree of the damage.

Such subjects include, for example, a patient with kidney disease requiring immunosuppressive treatment such as steroids or the like, a patient with severe kidney disease (renal failure or the like), a patient with kidney transplant, and the like.

Treatments for kidney disease include lifestyle improvement, dietary therapy, drug therapy, and the like, and can be selected in appropriate combinations according to the disease conditions. The method of the present invention makes it possible to conveniently and early grasp the progression of tubulointerstitial orders and the severity of kidney disease, thus enabling the selection of a treatment more suited to the patient's disease conditions. Furthermore, it is preferred because the use of steroids, which may cause side effects, can be selected with or without renal biopsy. Furthermore, it is preferred because the trends of progression of tubulointerstitial disorders and severity of kidney disease can be grasped and a treatment can be selected according to the trends by monitoring of urinary presepsin concentration.

Methods for detecting and grasping tubulointerstitial disorders and kidney disease can be appropriately designed and used by those skilled in the art based on known information.

The methods of measuring presepsin themselves are known, and can be performed by various known analysis methods of proteins, such as immunological analysis using antibodies, biochemical analysis such as electrophoresis or the like, and automatic analyzers for clinical laboratory test can also be used. Analytical methods using substances with properties similar to those of antibodies, such as RNA aptamers, are also part of the present invention.

For example, Japanese Patent No. 4040666 discloses methods of measuring human sCD14-ST, more specifically, sandwich EIA systems [Example 7-(1)] using combinations of a polyclonal antibody (S68 antibody) or a monoclonal antibody (F1146-17-2 antibody) prepared using a peptide consisting 16 amino acid residues of SEQ ID NO: 2 (S68 peptide described in Japanese Patent No. 4040666) as an antigen and an anti-CD14 antigen monoclonal antibody (such as F1031-8-3 antibody, F1106-13-3 antibody, or the like), which can be used in the method of the present invention.

Furthermore, as shown in the Examples below, the measurement of presepsin can also be performed by chemiluminescent enzyme immunoassay using magnetic particles, using an automated chemiluminescent immunoassay analyzer (PATHFAST; LSI Medience Corporation).

The sample used in the method of the present invention is not limited as long as it is possible to measure urinary presepsin. For example, urine collected from a subject can be used.

In the method of the present invention, a change in urinary presepsin concentration can be used as an indicator of tubulointerstitial disorders. Instead of urinary presepsin concentration, the urinary presepsin concentration/urinary creatinine (uP-SEP/Cr) value, which is the value obtained by compensating the urinary presepsin concentration with urinary creatinine, can be used. Compensation with urinary creatinine is preferred because it eliminates the influence of urine volume and the like. Unless otherwise specified, the urinary presepsin concentration or the urinary presepsin concentration/urinary creatinine value is referred to as the urinary presepsin value.

For example, as shown in Examples 5 and 6 below, the urinary presepsin values were higher in diseases with strong tubulointerstitial disorders and lower in diseases with weak tubulointerstitial disorders. In this way, in the method of the present invention, it is possible to judge that tubulointerstitial disorders are strong or that the disorders are in progress when the urinary presepsin value is high. Furthermore, in the method of the present invention, it is possible to judge that the severity of kidney disease is high or that the conditions are advanced when the urinary presepsin value is high. On the other hand, it is possible to judge that tubulointerstitial disorders are weak when the urinary presepsin value is low. On the other hand, it is possible to judge that the severity of kidney disease is low or that the conditions are improved when the urinary presepsin value is low. For example, if the urinary presepsin value in a sample is higher than the quantile (for example, median) in healthy subjects, it can be judged that tubulointerstitial disorders are strong or that the severity of kidney disease is high. It can also be performed by statistical methods such as Cox regression, logistic regression, or the like. Furthermore, the intensity of tubulointerstitial disorders or the severity of kidney disease can be grasped by the degree of urinary presepsin value. As the criteria for judgment, a "threshold value" prepared in advance can be used.

For example, if the urinary presepsin concentration is higher than approximately 990 pg/mL, it can be judged that tubulointerstitial disorders are strong, or that the subject is a patient with ANCA-associated vasculitis (AAV), diabetic nephropathy (DMN), lupus nephritis, or interstitial nephritis. Or, if the uP-SEP/Cr value is higher than approximately 1000 ng/gCr, it can be judged that tubulointerstitial disorders are strong, or that the subject is a patient with minimal change nephrotic syndrome (MCNS), membranous nephropathy, ANCA-associated vasculitis (AAV), diabetic nephropathy (DMN), lupus nephritis, or interstitial nephritis.

Furthermore, if the uP-SEP/Cr value is higher than approximately 900 ng/gCr, it can be judged that tubulointerstitial disorders are mild or higher, if the uP-SEP/Cr value is higher than approximately 1200 ng/gCr, it can be judged that tubulointerstitial disorders are moderate or higher, and if the uP-SEP/Cr value is higher than approximately 1800 ng/gCr, it can be judged that tubulointerstitial disorders are severe or higher.

In the method of the present invention, the threshold value of urinary presepsin value to grasp the intensity of tubulointerstitial disorders or the severity of kidney disease or to differentiate between tubulointerstitial disorders or kidney disease is expected to vary depending on various conditions, such as gender, age, or the like. However, those skilled in the art can determine the threshold value for judgment by appropriately selecting an appropriate population corresponding to the subjects and statistically processing the data obtained from the population. As such populations, a normal subject group, a non-tubulointerstitial disorder group, a tubulointerstitial disorder group, a severity group of each impairment of non-tubulointerstitial disorders, a severity group of each impairment of tubulointerstitial disorders, a type group of each impairment of non-tubulointerstitial disorders, a type group of each impairment of tubulointerstitial disorders, a non-kidney disease group, a kidney disease group, an extent (scale or the like) group of each disease condition of non-kidney disease, an extent (scale or the like) group of each disease condition of kidney disease, a type (scale or the like) group of each disease condition of non-kidney disease, a type (scale or the like) group of each disease condition of kidney disease, an acute group, a chronic group, a non-drug-induced group, a drug-induced group, or the like can be selected.

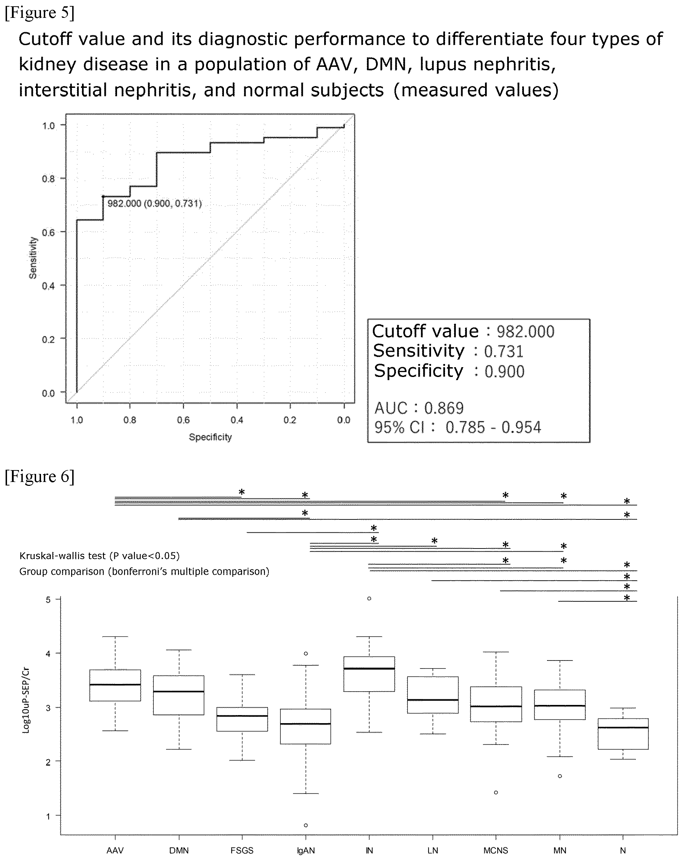

In Example 4 described below, an optimal cutoff value of 982 pg/mL (sensitivity: 73%, specificity: 90%, AUC: 0.869 (95% CI: 0.785-0.954)) is determined as the threshold value to detect disease conditions or diseases with tubulointerstitial disorders by performing ROC (receiver operating characteristic) analysis shown in Figure 5.

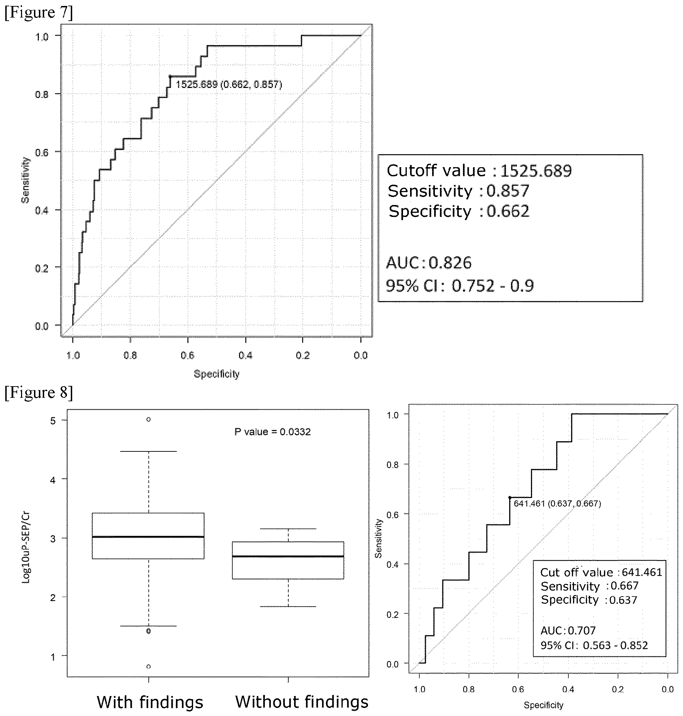

In Example 6 described below, an optimal cutoff value of 1525.689 (ng/gCr) (sensitivity: 85.7%, specificity: 66.2%, AUC: 0.826 (95% CI :0.752-0.9)) is determined as the threshold value to detect interstitial nephritis by performing ROC analysis shown in Figure 7.

In Example 7 described below, an optimal cutoff value of 641.461 (ng/gCr) (sensitivity: 66.7%, specificity: 63.7%, AUC: 0.707 (95% CI: 0.563-0.852)) is determined as the threshold value to detect renal disorders by performing ROC analysis shown in Figure 8.

In Example 8 described below, threshold values to detect tubulointerstitial disorders (inflammatory cell infiltration or tubular atrophy) are determined as follows by performing ROC analysis shown in Table 4.

About inflammatory cell infiltration, an optimal cut off value of 922.196 (ng/gCr) (sensitivity: 64.1%, specificity: 65.5%, AUC: 0.702 (95% CI: 0.653-0.750)) is determined as the threshold value to detect mild or higher, an optimal cut off value of 1210.588 (ng/gCr) (sensitivity: 69.8%, specificity: 66.5%, AUC: 0.665 (95% CI: 0.665-0.768)) is determined as the threshold value to detect moderate or higher, and an optimal cut off value of 1954.418 (ng/gCr) (sensitivity: 80.4%, specificity: 73.8%, AUC: 0.811 (95% CI: 0.751-0.871)) is determined as the threshold value to detect severe or higher.

About tubular atrophy, an optimal cut off value of 992.225 (ng/gCr) (sensitivity: 57.5%, specificity: 63.8%, AUC: 0.649 (95% CI: 0.595-0.704)) is determined as the threshold value to detect mild or higher, an optimal cut off value of 1210.588 (ng/gCr) (sensitivity: 65.2%, specificity: 65.8%, AUC: 0.706 (95% CI: 0.656-0.756)) is determined as the threshold value to detect moderate or higher, and an optimal cut off value of 1776.823 (ng/gCr) (sensitivity: 70.3%, specificity: 71.4%, AUC: 0.760 (95% CI: 0.700-0.820)) is determined as the threshold value to detect severe or higher.

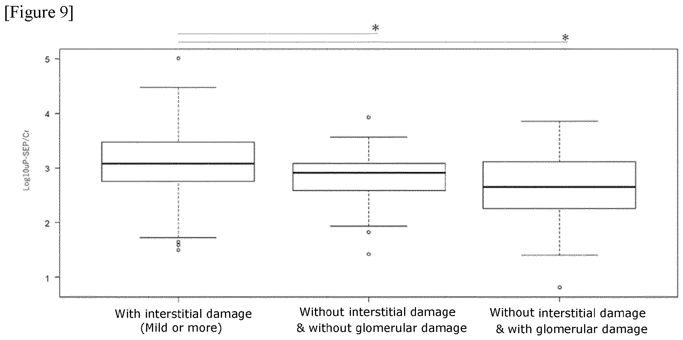

In Example 9 described below, an optimal cutoff value of 543.082 (ng/gCr) (sensitivity: 72.2%, specificity: 60.5%, AUC: 0.699 (95% CI: 0.575-0.823)) is determined as the threshold value to detect tubulointerstitial disorders in patients with IgA nephropathy by performing ROC analysis shown in Figure 9.

In the method of the present invention, tubulointerstitial disorders can be detected, with or without renal biopsy, by determining the threshold value for judgment and comparing the measured value of urinary presepsin in a sample with the threshold value for judgment.

In the method of the present invention, samples are collected at the stage of suspicion of tubulointerstitial disorders, at the stage of suspicion of progression of tubulointerstitial disorders, after treatment, or the like. In particular, the samples may be taken over time, after the suspicion of kidney disease with tubulointerstitial disorders arises, before judging whether to perform renal biopsy, or after the renal biopsy has been performed. For example, after the renal biopsy is performed, samples may be collected monthly, bimonthly, annually, or the like. In addition, since disease conditions can be evaluated conveniently instead of renal biopsy, the time of sampling can be appropriately selected as without burdening the patient, such as daily, weekly, monthly, bimonthly, annually, or the like.

As the first embodiment of the method of the present invention, in the early stage of tubulointerstitial disorders, it is possible to grasp the presence or absence and extent of the disorders with or without renal biopsy. The early stage of tubulointerstitial disorders includes the stage when suspicion of tubulointerstitial disorders arises. It is preferred because the severity can be grasped based on the grasp of renal damage in kidney disease with tubulointerstitial disorders by detecting tubulointerstitial disorders.

Furthermore, chronic tubulointerstitial disorders often progress without clinical symptoms, and the fact that the method of the present invention can detect tubulointerstitial disorders with or without renal biopsy is a very useful effect.

The stage when suspicion of tubulointerstitial disorders arises is, for example, the stage when clinical symptoms are not clearly observed. Chronic kidney disease (CKD) often progresses with little or no symptoms, and when symptoms surface, the disease is often severe, and therefore, it is very useful to detect tubulointerstitial disorders at an early stage. Since the method of the present invention can be used to detect tubulointerstitial disorders prior to the time of recognition of chronic kidney disease using conventional diagnostic guidelines, in a specific embodiment, the patient's disease state in early chronic kidney disease is confirmed further back in time than when the signs of chronic kidney disease are more clinically evident. In other words, it can be said that this is the stage when the doctor has the clinical suspicion of chronic kidney disease based on the clinical symptoms.

It also includes the stage of deciding whether or not renal biopsy should be performed, for example, to confirm tubulointerstitial disorders. Conventionally, renal biopsy is performed to evaluate renal damage in order to confirm chronic kidney disease, but in some cases, renal biopsy may have more risks than benefits for the patient, and a careful decision should be made as to whether or not to perform renal biopsy. The method of the present invention is preferred because tubulointerstitial disorders can be detected by urine samples and an appropriate time for renal biopsy can be selected conveniently and at an early stage.

Furthermore, since acute tubulointerstitial disorder occurs as acute kidney injury (AKI), it is a rapidly progressing condition, and treatment of the underlying disease is generally the mainstay of treatment. Therefore, tests such as renal biopsy or the like performed at rest to accurately identify tissue damage cannot be performed in patients with AKI. Since the method of the present invention can be used to detect tubulointerstitial disorders prior to the time of recognition of AKI without renal biopsy, in a specific embodiment, the patient's disease state in early acute kidney disease is confirmed further back in time than when the signs of acute kidney disease are more clinically evident. In other words, it can be said that this is the stage when the doctor has the clinical suspicion of acute kidney disease based on the clinical symptoms.

Furthermore, it also includes grasping the disease conditions of acute kidney injury from acute tubulointerstitial disorders, grasping its severity, and selecting the most appropriate treatment. In acute kidney injury, since it is difficult to accurately identify tissue damage by renal biopsy or the like, it is very preferable to detect tubulointerstitial disorders with a single biomarker. The method of the present invention is preferred because tubulointerstitial disorders can be detected by urine samples and a treatment can be selected conveniently and at an early stage.

As the second embodiment of the method of the present invention, at a later stage when tubulointerstitial disorders are recognized, with or without renal biopsy, tubulointerstitial disorders can be detected and the presence or absence and degree of the disorders can be grasped by measuring urinary presepsin. It is preferred because the severity can be grasped based on the grasp of renal damage in chronic kidney disease with tubulointerstitial disorders by detecting tubulointerstitial disorders. Similar to the first embodiment, the second embodiment of the method of the present invention is preferred because tubulointerstitial disorders can be detected by urine samples and an appropriate time for renal biopsy can be selected easily and at an early stage.

It also includes the measurement of urinary presepsin over time after renal biopsy. It is preferred because it reduces or eliminates the number of repeated renal biopsies.

It also includes the detection of kidney injury by measuring urinary presepsin over time in patients who do not undergo renal biopsy. Currently, renal biopsy cannot be performed when kidney disease is severe (for example, stage 3, stage 4, stage 5, or the like), when the kidney becomes fibrotic and hardened, when the kidney atrophies, when a specimen for renal biopsy cannot be taken due to single kidney, or when patients are at high risk. As a result, the opportunity to select a therapy tailored to the patient's conditions is lost due to the inability to perform renal biopsy. The method of the present invention makes it possible to grasp the onset of severe disease conveniently and at an early stage, and to select a therapy tailored to the patient's conditions. Furthermore, it is preferred because it can provide a basis for decision making to select a therapy with immunosuppressive agents such as steroids or the like, according to the evaluation of strong tubulointerstitial disorders or the like, with or without renal biopsy.

As the prognosis of AKI, it is preferred because the degree of kidney damage can be diagnosed, and the progression to CKD can be grasped conveniently and at an early stage.

As the third embodiment of the method of the present invention, the severity of kidney disease due to the effect of treatment can be grasped. The grasp of the severity due to the effect of treatment is not limited, but monitoring the effect of treatment is exemplified described below. For example, when samples are collected before treatment after a patient comes to the hospital, after treatment, and at the time when the effect of treatment appears, and if the predetermined threshold value is exceeded despite treatment, the progression of severity can be grasped due to the lack of effect of treatment. On the other hand, if the patient's disease conditions have already exceeded the threshold value at the time of admission and then falls below the threshold value as a result of treatment, it can be grasped that the treatment is effective and the severity is improved.

It is preferred that the severity can be grasped by the method of the present invention, with or without renal biopsy. Especially in the case of acute kidney injury, the current diagnostic guidelines do not allow for differentiation of tissue damage by renal biopsy or the like, thus limiting the choice of treatment. Therefore, it is very preferable that the disease conditions of acute kidney injury can be grasped conveniently by the method of the present invention.

The kit of the present invention can be used to carry out the method of the present invention, and comprises:
(a) an antibody specific for presepsin,
(b) standard data correlating the presepsin value in urine with the degree of kidney damage, and
(c) an instruction manual.

The kit of the present invention can be used to carry out the method of the present invention, and comprises:
(a) an antibody specific for presepsin,
(b) standard data correlating the presepsin value in urine with the severity of kidney disease, and
(c) an instruction manual.

The antibodies used in the kit of the present invention can be either monoclonal or polyclonal antibodies. Furthermore, antibody fragments that retain specific binding ability to presepsin, such as Fab, Fab', F(ab')₂, or Fv, can be used in the kit.

Furthermore, the antibodies can be used in the kit as they are, or in a suitable form based on the immunological method to be used, for example, immobilized on latex carriers if latex agglutination immunoassay is used, immobilized on magnetic particles if high-sensitivity measurement method using magnetic particles or the like is used, immobilized on a substrate if a substrate for immunochromatography or the like is used, or labeled with a labeling substance (for example, enzyme, fluorescent substance, chemiluminescent substance, radioisotope, biotin, avidin) if necessary.

The standard data included in the kit of the present invention are not limited as long as they show the correlation between the presepsin value in urine and the degree of kidney damage or the severity of kidney disease. For example, the threshold value for judgment, original data or statistical processing data for calculating the threshold value, or the like can be exemplified. The standard data may be described in the instruction manual, or separately attached as a data sheet. The attached document may be in the form of paper, electronic media such as CD-ROMs or the like, or a download from websites or the like.

The instruction manual included in the kit of the present invention is not limited as long as it refers to the relationship between the presepsin value in urine and the degree of kidney damage or the severity of kidney disease. In addition to the aforementioned references, it can include, for example, a description of the procedure for conducting immunological measurements using the kit of the present invention, a description of the procedure for predicting prognosis based on the obtained measurement values, precautions for storage and handling of the kit itself, and the like.

### EXAMPLES

The present invention is not limited to the following embodiments and Examples. Without departing from the claims, various variations are also included in the present invention to the extent conceivable by those skilled in the art.

### «Example 1: Measurement of presepsin in urine by CLEIA method using anti-presepsin antibodies»

The measurement of presepsin in urine was carried out by modifying the method described in Example 7-(1) of Japanese Patent No. 4040666, in which presepsin in blood can be measured. Specifically, a polyclonal antibody (S68 antibody) labeled with alkaline phosphatase (ALP) and a monoclonal antibody (F1031-8-3 antibody) immobilized on magnetic particles (manufactured by JSR) were used, and the measurement was carried out using an automated chemiluminescent immunoassay analyzer (PATHFAST; manufactured by LSI Medience Corporation). The polyclonal antibody (S68 antibody) labeled with alkaline phosphatase (ALP) was prepared by preparing a Fab' fraction of the polyclonal antibody (S68 antibody) and linking the same with ALP by a maleimide method. CPD-star (manufactured by Applied Biosystems) was used as a luminescent substrate. These reagents are referred to as a presepsin PATHFAST kit in the following examples.

The measurement was carried out as follows. A sample was reacted with the antibody immobilized on magnetic particles and the ALP-labeled antibody to form a complex composed of presepsin contained in the sample and both antibodies. The complex was collected by a magnetic body to remove the unbound ALP-labeled antibody. The luminescent substrate was added to detect the amount of luminescence as the amount of presepsin.

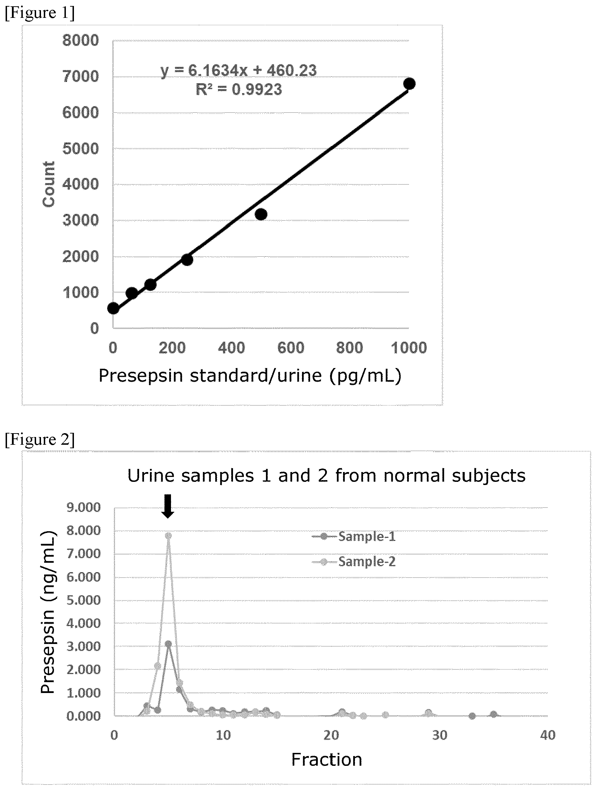

Figure 1 shows a calibration curve prepared by the above measurement method, in which a presepsin standard (manufactured by LSI Medience Corporation) was added to a urine sample from a normal subject and sequentially diluted two-fold with the same urine. It was shown that presepsin in urine can be measured in the same manner using the method for measuring presepsin in blood.

### «Example 2: Specific detection of presepsin in urine»

In order to confirm whether presepsin present in human urine was actually detected, urine was concentrated, the sample was fractionated by gel filtration, and each fraction was tested by ELISA (enzyme-linked immunosorbent assay) using anti-presepsin antibodies.

First, each urine (5 mL) from two normal subjects was concentrated approximately 10-fold using a centrifugal filter (Amicon Ultra-15, 3000MNWL, Merck). Each of the concentrated samples (0.4 mL) was applied to a gel filtration column (Superdex 75 Increase 10/300; manufactured by GE Healthcare Japan) attached to a high-performance liquid chromatography system (SHIMADZU LC-10AT/SPD-6A/UV; manufactured by Shimadzu Corporation), and was eluted with Tris-buffered saline pH 7.6 containing 0.05% Tween-20 (manufactured by Sigma-Aldrich)(hereinafter referred to as T-TBS). Forty fractions per sample were obtained with 0.4 mL/fraction of each eluate. Next, 0.4 mL of a presepsin standard (manufactured by LSI Medience Corporation) was applied to the same system and fractionated in the same manner.

Each of the obtained fractions was measured by ELISA using the anti-presepsin antibodies described in Example 1 (presepsin ELISA). The sandwich ELISA constructed using the anti-presepsin antibodies was carried out as follows. Specifically, an F1106-13-3 antibody diluted with 0.05 mol/L of a Tris buffer solution (TBS, pH7.6) was diluted to 5 µg/mL, and added to an immunoplate (Maxisorp; NUNC) at 100 µL/well. After the reaction was carried out at 4°C overnight, the wells were washed three times with
Tris buffer saline containing 0.05% Tween-20 (T-TBS), and blocked with 200 µL/well of TBS containing 0.1% StabilGuard (manufactured by Surmodics) and 0.1% Tween-20. Next, a standard dilution series of human presepsin standard (manufactured by LSI Medience Corporation) used as a standard was prepared using 0.1% bovine serum albumin (Sigma-Aldrich)/TBS. Each fraction fractionated was measured undiluted. Each fraction was added at 100 µL/well, reacted for 1 hour at room temperature, and then washed three times in the same manner. Next, a prepared biotin-labeled S68 antibody (prepared according to a manual using an EZ-Link NHS-LC-Biotin (manufactured by Thermo Fisher) reagent) was diluted to 1 µg/mL with T-TBS and added to each well at 50 µL/well. After the reaction was carried out at room temperature for 1 hour, the wells were washed three times in the same manner. Next, AMDEX streptavidin-conjugated horseradish peroxidase (manufactured by GE Healthcare) was diluted 7000-fold with T-TBS, and added to each well at 50 µL/well. After the reaction was carried out at room temperature for 30 minutes, the wells were washed four times in the same manner, and a 3,3',5,5'-tetramethylbenzidine (TMB) solution (manufactured by Sigma-Aldrich) was added to each well at 50 µL/well. After the reaction was carried out at room temperature for approximately 20 minutes, the reaction was stopped with a 1 mol/L sulfuric acid solution (manufactured by FUJIFILM Wako). A plate spectrophotometer (EL312e; manufactured by BIO-TEK INSTRUMENTS) was used to measure absorbance at 450 nm (subwavelength 630 nm).

In Figure 2, the presepsin concentrations obtained by the presepsin ELISA of urine from two normal subjects are shown on the vertical axis, and each fraction number is shown on the horizontal axis. Furthermore, the position where the presepsin standard was observed are indicated by the arrow. In the urine of normal subjects, fractions with a molecular weight equivalent to that of the presepsin standard was identified, and it was confirmed that presepsin in urine was specifically detected by the ELISA using the anti-presepsin antibodies.

### «Example 3: Collection of urine samples and renal biopsy»

From patients who were judged to require a renal biopsy based on the results of serum creatinine, eGFR level, urinary protein, urinary microalbumin, urinary occult blood, and the like and had renal biopsy performed between March and December 2020, consent forms for Nagoya-Kidney Disease Registry (N-KDR) Study (Nagoya University Ethics Committee Approval No. 2010-1135-7) were obtained, and patient's spot urine was collected on the day before the renal biopsy. At affiliated hospitals, urine was collected within 24 hours after refrigeration and frozen at -80°C after aliquoting. Urine samples collected at Nagoya University Hospital were kept refrigerated, aliquoted within 6 hours, and frozen at -80°C.

Renal biopsy was performed as follows. The patient was placed in the prone position, and the site to be punctured was cleaned with an iodine-based disinfectant. Next, to avoid complications of renal biopsy (bleeding around the kidney or the like), the puncture site was selected mainly at the inferolateral part of the lower pole of the kidney where enough renal cortical tissue could be obtained. The skin surface of the left back was scanned by the probe of an ultrasonic device (3.0 to 3.5 MHz) and marked with a marker where the puncture needle would be inserted, and anesthesia with a local anesthetic was administered along the skin where the puncture needle would be inserted and the puncture needle insertion route. The skin was incised before puncturing the biopsy needle, and an automated biopsy device was operated in conjunction with a 14 to 18G automated biopsy device to collect renal parenchymal tissue. The collected renal tissue was transferred to saline-soaked gauze to prevent drying until each fixation. Similarly, tissue sections were collected 2 to 3 times. After the puncture, the patient's back was manually compressed to stop the bleeding.

The collected tissues were cut crosswise using a FEATHER double-edged razors. Next, four types of staining were carried out for renal biopsy pathological diagnosis, and one section of each was prepared. Optical specimens were fixed in MaskedForm fixative from 3 hours to overnight, dehydrated, defatted, and permeated with paraffin, and then paraffin-embedded blocks were prepared. Staining was carried out according to conventional methods, using a combination of various staining methods such as HE staining, PAS staining, PAM staining, Elastica-Masson staining, and the like. Optical microscopy allowed us to observe the glomeruli, renal tubules, blood vessels, and the like as a whole, providing basic histological information. The specimens for electron microscopy were fixed in 2.5% glutaraldehyde for 2 hours to overnight (4°C) and washed three times with 0.1 mol/L PB buffer for 10 minutes. Next, the specimens were fixed in 2% osmium acid fixing solution for 2 hours and dehydrated by changing the ethanol concentration from 50% to 100%. The obtained sections were magnified up to approximately 20,000 times by an electron microscopy to confirm the structure of the glomeruli and renal tubules, or the internal structure of their constituent cells, the deposits that cause nephritis, and the like. Furthermore, frozen tissue sections were prepared. In the fluorescent antibody test, the presence and location of depositions of immunoglobulins such as IgG, IgA, IgM, and the like and complements such as C3, C1q, and the like were observed. Specifically, fluorescent-labeled antibodies that react specifically with target antigens were reacted. After the reaction, the molecules to which the antibodies bind were observed using a fluorescence microscope. Three tests (i.e., optical microscopy test, electron microscopy test, and fluorescent antibody test) were combined to diagnose the cause of kidney disease, and the degree of inflammatory cell infiltration and tubular atrophy were calculated. In the calculation, the area of each lesion was visually observed with Elastica-Masson staining and classified into 4 levels: Minimal (<5%), Mild (5-25%), Moderate (26-50%), and Severe (>50%) in increments of 25%. The degree of each lesion was evaluated by the consensus of at least three nephrologists.

### «Example 4: Comparison of presepsin in urine and presepsin in blood»

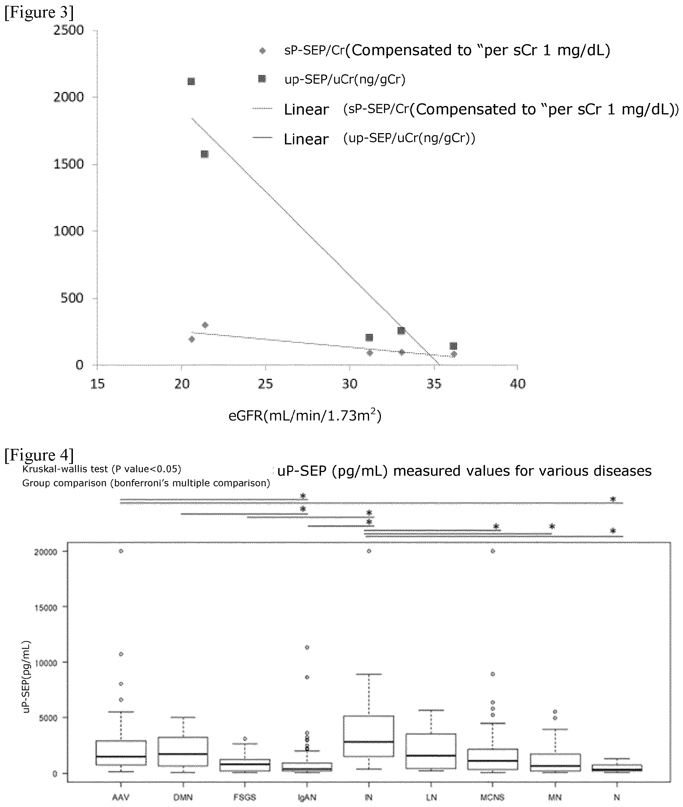

Since presepsin is elevated in septic patients, it is used as a marker for the diagnosis of sepsis. It has been reported that the GFR classification of CKD patients shows no effect of renal failure up to G2, but a gradual increase from G3. As a cause, it is clear from studies in dogs that presepsin is excreted in urine by renal metabolism (Patent literature 3), and it has been reported that the concentration of presepsin in blood increases with decreased renal function (increased GFR stage classification) (Non-Patent literature 2). Miyoshi et al. (Non-Patent literature 3) have reported that the effect of reduced renal function can be eliminated by compensating the presepsin value in blood with the blood creatinine value. Therefore, the present inventors analyzed the relationship between blood presepsin and urinary presepsin in patients with renal failure without infection after creatinine compensation. The present inventors compared blood presepsin (per 1 mg/dL of creatinine) and urinary presepsin (uP-SEP/gCr) in 5 CKD patients with preserved plasma and urine, respectively. As a result, urinary presepsin as well as blood presepsin showed a negative correlation with GFR stage classification. While blood presepsin showed a trend of gradual increase with a decrease in eGFR value, the increase was small and not at a level where the disease state was graspable. In addition, it was recognized that the results only support the report of Non-Patent literature 3. On the other hand, it has been revealed that urinary presepsin responds sensitively to the decrease in eGFR value, and its rate of increase is also high. These results indicate that urinary presepsin may reflect the renal state more sensitively than blood presepsin, and it has been revealed that urinary presepsin is an excellent marker for grasping the renal state (Figure 3).

Immunohistological examination of renal biopsy tissues from nephropathy patients shows that the number of macrophages in the glomeruli and interstitium is clearly increased compared to normal renal tissues. Macrophages have a variety of functions such as antigen presentation, phagocytosis, production of cytokines and growth factors, and the like, and play an important role in biological defense such as immune responses and the like. On the other hand, activated macrophages are an important factor in the development of tissue injury by locally releasing inflammatory cytokines, proteases, and nitric oxide (NO)(SHIKATA, Kenichi, Jpn J Nephrol, 2007;49(5):474-480). This indicates that the increase in inflammatory cells is closely related to renal damage, and the more sensitive elevation of presepsin in urine than in blood may reflect the result that presepsin is produced at the site of such damage and is transferred into urine through the disrupted and leaky renal tubules. Although it has been known that presepsin is elevated with phagocytosis of microorganisms in infectious diseases, it was not known that presepsin is also produced by tissue damage such as renal injury, and its production outside the local infection was a surprising finding.

### «Example 5: Concentrations of urinary presepsin (urinary P-SEP) in various kidney diseases»

The present inventors have studied whether urinary presepsin better reflects renal conditions and found that urinary presepsin is elevated in kidney diseases, in particular, in interstitial nephritis. Therefore, in order to clarify what kinds of kidney diseases are associated with urinary presepsin, we investigated the relationship between the concentration of urinary presepsin and chronic kidney disease (CKD) in 463 urine samples from patients collected in the Registry Study. All 463 patients were subject to renal biopsy, and a definitive diagnosis of each kidney disease was made. The 463 patients included 38 patients with ANCA-associated vasculitis (AAV), 26 patients with diabetic nephropathy (DMN), 23 patients with glomerulosclerosis (FSGS), 106 patients with IgA nephropathy, 53 patients with nephrotic syndrome (MCNS), 41 patients with membranous nephropathy (MN), 12 patients with lupus nephritis, 28 patients with interstitial nephritis, and others. Table 1 shows the background of the patients. Urine samples were also collected from 10 normal volunteers.

**[Table 1]**

| Background of patients | |
|---|---|
| Total patients | 463 patients |
| Gender ratio | Male 263/Female 200 |
| Age | 55.7±19.1 years old |
| BUN | 25.7±21.9mg/dL |
| Urinary creatinine (Cr) | 1.81±2.1mg/dL (Mean ± standard deviation) |
| | 1.1(0.79-1.78) mg/dL* |
| Urinary protein creatinine ratio (UP) | 2.03(0.83-5.70) g/gCr* |

| | |
|---|---|
| *: (Median, 25-75th percentile) | |

The concentrations of urinary presepsin were measured using an automated chemiluminescent immunoassay analyzer (PATHFAST). Specifically, the presepsin PATHFAST kit of Example 1 was provided, 100 µL of each urine sample was dispensed into the sample port of the reagent cartridge, and the reagent cartridge was placed on the analyzer. The analyzer was set up according to the manual, and the concentrations of urinary presepsin were measured. The results of urinary presepsin concentration measurements (measured values) for each disease are shown in Figure 4. The mean ± standard deviation and median (quartiles 25%, 75%) for each disease are shown in Table 2.

**[Table 2]**

| Urinary presepsin in kidney disease patients (measured values without urinary creatinine compensation) | | | |
|---|---|---|---|
| Disease (n) | Mean ± standard deviation (pg/mL) | Median (Quartiles 25%-75%) (pg/mL) | P value (Comparison with normal subjects) |
| Normal subjects (10) | 470.7±400.5 | 312.0 (200.8-641.0) | |
| AAV (38) | 2395.6±3476.8 | 1502.0 (726.0-2318.0) | 0.0005* |
| DMN (26) | 2013.2±1474.8 | 1705.0 (707.3-3247.3) | 0.0042* |
| FSGS (23) | 920.6±864.4 | 771.0 (190.5-1242.0) | 0.3084 |
| IgA nephropathy (106) | 848.7±1500.5 | 355.5 (173.3-939.3) | 0.7529 |
| MCNS (53) | 1998.4±3161.8 | 1100.0 (299.0-2166.0) | 0.0626 |
| MN (41) | 1156.7±1315.4 | 627.0 (186.0-1717.0) | 0.2355 |
| Lupus nephritis (12) | 2139.0±1960.3 | 1606.0 (460.5-2950.0) | 0.0122* |
| Interstitial nephritis (28) | 4807.6±5782.6 | 2831.5 (1510.8-4839.3) | <0.0001 * |
| Total patients (n=463) | 1670.6±2838.9 | 795.0 (244.0-1994.0) | |

The concentrations of urinary presepsin were found to be low in normal subjects (control group) and significantly elevated in patients with various renal disorders (Mann-Whitney U test, *; P value<0.05). In addition, the concentrations of urinary presepsin were lower in IgA nephropathy, FSGS, MN, and MCNS, which are glomerular diseases and presumed to have relatively low levels of interstitial damage and renal tubular damage, and significantly elevated in interstitial nephritis, AAV, DMN, and lupus nephritis, which have high levels of interstitial damage and renal tubular damage, compared to normal subjects or IgA nephropathy. These results indicate that the concentration of urinary presepsin is a detectable marker of the degree of damage, in particular, in the interstitium and renal tubules (tubulointerstitial disorder).

By using, as a population, a group of AAV, DMN, lupus nephritis, and interstitial nephritis patients, in which significant differences were found against normal subjects, plus normal subjects, the results of ROC curve (receiver-operating characteristic curve) analysis for these four diseases are shown in Figure 5.

As a result, the sensitivity, the specificity, and the AUC were 73.1%, 90.0%, and 0.869 (95%CI 0.785-0.954), respectively, at the cutoff value of 982 pg/mL. It was also revealed from the results that diseases with high possibility of having renal disorders (in particular, tubulointerstitial disorder) could be clearly distinguished.

Conventionally, it has been known that presepsin can be used as an indicator of infectious diseases (in particular, sepsis), because it is induced and produced by macrophages generated by bacterial infection or the like. However, as shown in this Example, it was surprising that urinary presepsin could reflect inflammation independently of infection, and furthermore, correlated with inflammation in the renal interstitium, not with glomerular inflammation.

### «Example 6: Compensation of presepsin in urine by urinary creatinine»

When markers in spot urine are measured, compensation by urinary creatinine (Cr) is used to eliminate the effect of fluctuation of the concentration in samples due to the volume of urine. Therefore, in the present study, a value (uP-SEP/Cr) obtained by dividing the concentration of urinary presepsin (uP-SEP) by urinary creatinine (Cr) was also used for evaluation. Figure 6 shows the values of uP-SEP/Cr in various kidney diseases as Log10 uP-SEP/Cr (ng/gCr), and group comparisons between these diseases were carried out by bonferroni's multiple comparison and shown by Kruskal-wallis test (*: P value<0.05).

The results showed that the uP-SEP/Cr values were significantly higher, particularly, in interstitial nephritis, which has high levels of interstitial damage and renal tubular damage, and that significant differences were observed between diseases and increases in the uP-SEP/Cr values were lower in focal glomerulosclerosis and IgA nephropathy, which are glomerular diseases and presumed to have relatively low levels of interstitial damage and renal tubular damage, and it was revealed that the uP-SEP/Cr values were consistent with interstitial damage regardless of diseases. It was also shown to be a marker specific to the degree of renal damage (especially, tubulointerstitial disorder), rather than reflecting the concentration of urinary presepsin or the like due to decreased urine volume or the like associated with the disease state.

Next, the sensitivity and specificity of uP-SEP/Cr values in detecting interstitial nephritis were calculated. Furthermore, the results of the receiver-operating characteristic curve (ROC curve) analysis are shown in Figure 7. The cutoff value was set by ROC curve analysis as follows. That is to say, the cutoff value to distinguish the progression of damage to the interstitium is indicated, since renal failure that causes damage to the interstitium is severe. Compared to the uP-SEP/Cr values in patients with renal disorders other than interstitial nephritis, the cutoff value for detection of interstitial nephritis was calculated to be 1525.689 (ng/gCr). The sensitivity and specificity of the urinary presepsin marker were 85.7% and 66.2%, respectively. The AUC of the ROC curve was 0.826, indicating that the ROC curve is highly useful as a single biomarker to detect interstitial nephritis, which has never been used before.

In the Examples described below, the results for uP-SEP/Cr values are shown, but similar results were obtained for uP-SEP values.

### «Example 7: Renal biopsy findings and uP-SEP/Cr values »

The results of renal biopsies of all patients were compared between those with and without findings of renal disorders. Figure 8 shows the values of uP-SEP/Cr in each group as Log10 uP-SEP/Cr (ng/gCr), and group comparisons between the groups were carried out by bonferroni's multiple comparison and shown by Kruskal-wallis test (*: P value<0.05). As a result, the uP-SEP/Cr values were significantly higher in patients with findings of renal disorders, i.e., kidney damages such as interstitial damage, glomerular damage, and the like (Figure 8, P=0.0332). The results of ROC analysis showed a cutoff value of 641.461 (ng/gCr) with a sensitivity of 66.7%, specificity of 63.7%, and AUC of 0.707 (95% CI: 0.563-0.852), indicating that the urinary presepsin value is associated with patients with renal biopsy findings of renal disorders.

Furthermore, the present inventors analyzed the patients who were subjected to renal biopsy separately for interstitial damage and glomerular damage. Patients were classified into three groups: those with interstitial damage (Mild or more), those without interstitial damage + without glomerular damage, and those without interstitial damage + with glomerular damage, and compared with uP-SEP/Cr values. As shown in Figure 9, the Kruskal-Wallis test (P value < 0.05) with bonferroni multiple comparisons between groups showed that the groups with and without interstitial damage (including with glomerular damage) could be distinguished with significant differences. The uP-SEP/Cr values in each group are expressed as Log10 uP-SEP/Cr (ng/gCr). In the group without interstitial damage, there was no significant difference between the groups with glomerular damage and without glomerular damage. This indicates that urinary presepsin can specifically diagnose interstitial disorders and can differentiate them from glomerular disorders.

### «Example 8: Differentiation of degree of renal disorders by findings of renal biopsy and uP-SEP/Cr values»

The tissues surrounding the glomeruli and renal tubules in the kidney are called the interstitium, and interstitial nephritis is a disease in which renal function is impaired due to inflammation of the interstitium. Furthermore, inflammation often occurs not only in the interstitium but also in the renal tubules, and inflammatory cell infiltration in the interstitium and tubular atrophy observed in renal findings are important findings for the degree of renal damage in interstitial nephritis. The patients were classified into Minimal (<5%), Mild (<5-25%), Moderate (26-50%), and Severe (>50%) according to the degree of damage based on the imaging of renal biopsies. Significant differences between groups were tested for Minimal using Bonferroni's multiple comparisons, and significant differences were calculated using Kruskal-Wallis samples (P value <0.001).

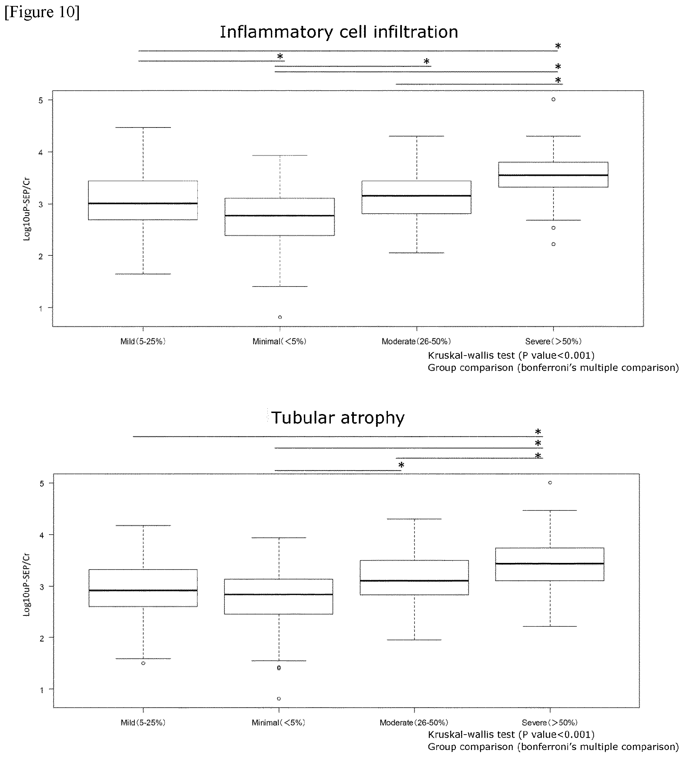

As a result, both inflammatory cell infiltration and the degree of tubular atrophy were associated with an increase in the degree of damage and the uP-SEP/Cr values, and significant differences were obtained between each group (Figure 10). In the inflammatory cell infiltration and the degree of tubular atrophy shown in Figure 10, there was a significant difference between Minimal and Mild in inflammatory cell infiltration, but not in tubular atrophy. Inflammatory cell infiltration precedes tubular atrophy. Thus, the degree of increase in uP-SEP/Cr values among the groups indicated that the increase was more rapid in inflammatory cell infiltration, which is useful for understanding the degree of progression of tubulointerstitial disorders in the acute phase, and monitoring uP-SEP/Cr values can be used to initiate treatment and to determine treatment efficacy.

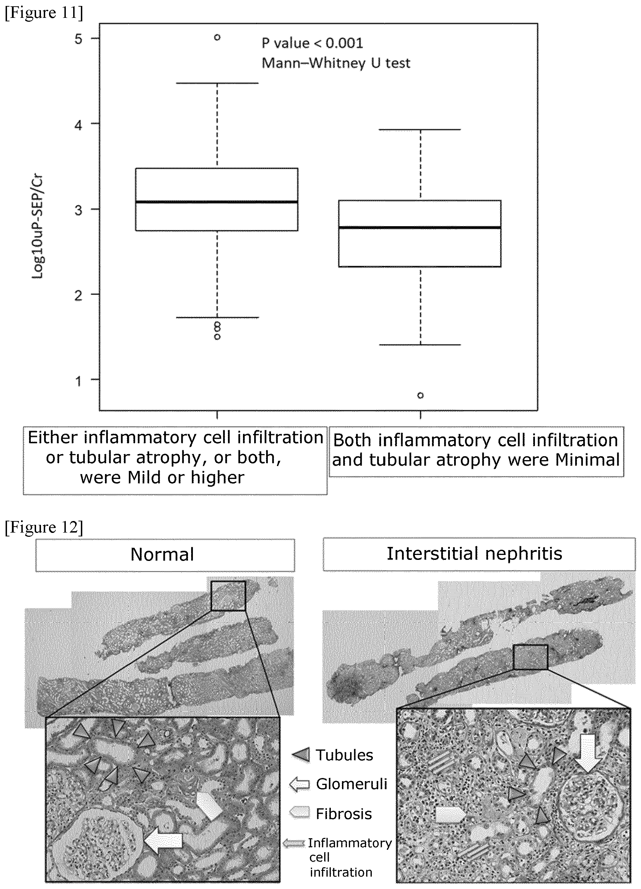

In order to confirm the relationship between the inflammatory cell infiltration and the degree of tubular atrophy, which indicate interstitial disorders, and uP-SEP/Cr values more clearly, a significant difference test was performed for the group of patients in which either inflammatory cell infiltration or tubular atrophy, or both, were Mild or higher and the group in which both inflammatory cell infiltration and tubular atrophy were Minimal (Figure 11). The results showed that uP-SEP/Cr values were higher when either inflammatory cell infiltration or tubular atrophy was Mild or higher (p<0.001), indicating that urinary presepsin can be used to determine the presence of interstitial renal disorders.

Next, Table 3 shows the results of the significant difference test between the groups in the case of Minimal, using bonferroni's multiple comparisons, and calculating the significant difference with Kruskal-Wallis samples (P value<0.001). The results show that the inflammatory cell infiltration and the degree of tubular atrophy (Mild to Severe) increases with each 100 ng/gCr increase in uP-SEP/Cr, and can be distinguished with significant differences.

**[Table 3]**

| Significant difference test of uP-SEP/Cr values in inflammatory cell infiltration and tubular atrophy (Vs: minimal)(With each 100 ng/gCr increase) | | | |
|---|---|---|---|
| | Odds ratio | 95%CI | P value |
| Inflammatory cell infiltration | | | |
| Mild | 1.040 | 1.020-1.05 | <0.001 |
| Moderate | 1.020 | 1.010-1.030 | <0.001 |
| Severe | 1.020 | 1.010-1.030 | <0.001 |
| Tubular atrophy | | | |
| Mild | 1.03 | 1.01-1.04 | <0.001 |
| Modereta | 1.020 | 1.010-1.030 | <0.001 |
| Severe | 1.020 | 1.010-1.030 | <0.001 |

Table 4 shows the cutoff values, sensitivity, specificity, and AUC obtained from the ROC analysis. These results indicate that the cutoff values do not differ between the degree of inflammatory cell infiltration and the degree of tubular atrophy, and that the degree of inflammatory cell infiltration and the degree of tubular atrophy cannot be distinguished, but the progress of tubulointerstitial disorders can be judged by the degree of its increase during the acute stage. The cutoff values reflected the sum of tubular atrophy (chronic phase symptoms) and inflammatory cell infiltration (acute phase symptoms).

**[Table 4]**

| ROC analysis results of inflammatory cell infiltration and tubular atrophy degrees (Cutoff value, sensitivity, specificity, and AUC (95% CI) in each group are shown (Vs: minimal)) | | | | |
|---|---|---|---|---|
| | Cutoff value (ng/gCr) | Sensitivity (%) | Specificity (%) | AUC (95%CI) |
| Inflammatory cell infiltration | | | | |
| Mild or more | 922.196 | 64.1 | 65.9 | 0.702 (0.653-0.750) |
| Moderate or more | 1210.588 | 69.8 | 66.5 | 0.665 (0.665-0.768) |
| Severe or more | 1954.418 | 80.4 | 73.8 | 0.811 (0.751-0.871) |

| Tubular atrophy | | | | |
|---|---|---|---|---|
| Mild or more | 992.225 | 57.5 | 63.8 | 0.649 (0.595-0.704) |
| Moderate or more | 1210.588 | 65.2 | 65.8 | 0.706 (0.656-0.756) |
| Severe or more | 1776.823 | 70.3 | 71.4 | 0.760 (0.700-0.820) |

As described above, the uP-SEP/Cr value was shown to be a sensitive biomarker for the progression of tubulointerstitial disorders and also a useful marker reflecting the overall degree of damage of tubulointerstitial disorders, and was found to be a useful complement to renal biopsy.

The images of renal biopsy used for the judgement are shown in Figure 12 left (normal case: inflammatory cell infiltration Minimal, tubular atrophy Minimal) and Figure 12 right (interstitial nephritis case: inflammatory cell infiltration Severe, tubular atrophy Severe).

In the normal case, the gap between renal tubules is very small and the tubules are adjacent to each other. In addition, fibrosis, if present, is minimal. However, in the case of interstitial nephritis, inflammatory cell infiltration in the interstitium, fibrosis, and tubular atrophy are seen, and the interstitial space is enlarged.

### «Example 9: Relationship between tubular atrophy and interstitial fibrosis (T-score) and uP-SEP/Cr values in the Oxford classification of IgA nephropathy»

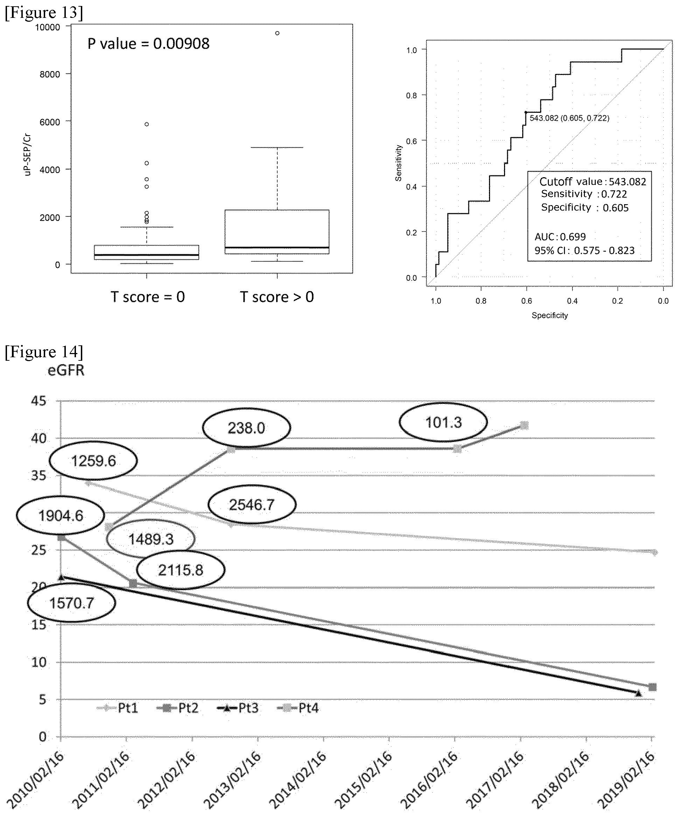

A renal biopsy is necessary to confirm the diagnosis of IgA nephropathy. Renal biopsy is useful not only for definitive diagnosis, but also for prognosis prediction and treatment selection based on tissue activity and severity. Renal biopsy shows cell proliferation in the mesangial area of the glomeruli and deposition of IgA and complement C3, a type of immune component, in the glomerular mesangial area. Therefore, in order to suppress the production of abnormal IgA, tonsillectomy is performed and combined with steroid pulse therapy to suppress glomerular inflammation (tonsillectomy pulse therapy). However, it has been reported that about 40% of patients with IgA nephropathy develop renal failure within 20 years, presumably due to chronic inflammation of the glomeruli and gradual hardening of the glomeruli, resulting in deterioration of renal function. The T-score in the Oxford classification is based on the tubular atrophy or the percentage of interstitial fibrosis in the cortex, and is expected to accurately predict the progression of IgA nephropathy and contribute to improved prognosis. Therefore, the present inventors examined whether uP-SEP/Cr values are related to T-score. The present inventors classified 106 patients with IgA nephropathy whose T-score was calculated using a cutoff value of T-score>0. The significance difference test was performed by Bonferroni's multiple comparisons using the Kruskal-Wallis samples (P value<0.001), and an increase was observed in patients having a higher T-score (Fig. 13, P value=0.00908). ROC analysis showed that the cutoff value was 543.082 (ng/gCr), with sensitivity of 72.2%, specificity of 60.5%, and AUC of 0.699 (95% CI: 0.575-0.823). The results indicate that uP-SEP/Cr values can detect tubulointerstitial disorders in patients with IgA nephropathy, and that uP-SEP/Cr values are useful for the diagnosis of severe IgA nephropathy.

### «Example 10: Evaluation of uP-SEP/Cr values in patients with chronic kidney disease (CKD)»

The present inventors analyzed the relationship between patients' uP-SEP/Cr values and subsequent renal function in 5 CKD patients with diabetic nephropathy, nephrosclerosis, and the like. Table 5 shows the patient background and uP-SEP/Cr values of the five patients and the subsequent rate of decrease in eGFR values.

**[Table 5]**

| Background of CKD patients and treatment history | | | | |
|---|---|---|---|---|
| Case number | Diagnosis | eGFR | uP-SEP/Cr (ng/gCr) | Rate of decline in eGFR (%/year) |
| 1 | Single kidney, Nephrosclerosis | 33.1 | 249.8 | 6.2 |
| 2 | Nephrosclerosis | 31.2 | 201.7 | 3.5 |
| 3 | Unknown | 36.2 | 139.8 | 3.3 |
| 4 | Single kidney, DM nephropathy | 20.6 | 2115.8 | 8.5 |
| 5 | Single kidney | 21.4 | 1570.7 | 8.2 |

In 5 patients with CKD, the uP-SEP/Cr values tended to be higher in patients with a high rate of eGFR decline and lower in patients with a low rate of eGFR decline. These results indicate that uP-SEP/Cr values reflect the degree of damage of renal function in CKD patients, even at the stage when renal biopsy cannot or will not be performed. According to the eGFR decline rate, patients with higher uP-SEP/Cr values showed a higher degree of renal dysfunction. The results showed that the uP-SEP/Cr value was associated with the degree of damage of renal function in patients and could reflect prognosis. Therefore, the uP-SEP/Cr value was shown to be useful in diagnosing the degree of severity in CKD patients.

### «Example 11: Treatment progress of chronic kidney disease (CKD) patients and patient monitoring using uP-SEP/Cr values»

Renal biopsy is a very important test for identifying the cause of CKD and formulating treatment plans. However, there are many patients for whom renal biopsy cannot be performed, such as those with a strong bleeding tendency, those with only single kidney, those with renal atrophy, those who do not wish to undergo renal biopsy, or the like. In that case, the cause must be estimated and treated, but there are disadvantages such as the inability to choose strong steroid treatment or the like if a definitive diagnosis cannot be made. The above results indicate that the uP-SEP/Cr value is a marker of tubulointerstitial disorders, and it is expected that the uP-SEP/Cr value reflects changes in the patient's conditions when monitoring the CKD patient. Therefore, the present inventors observed changes in eGFR and uP-SEP/Cr values over time for approximately 9 years from 2010 to 2019 in 4 CKD patients who did not undergo renal biopsy.

The results are shown in Figure 14. In case 1 (Pt1) and case 2 (Pt2), there was a correlation between a decrease in eGFR and an increase in uP-SEP/Cr values. Case 4 (Pt4) had a high uP-SEP/Cr value of 1489.3 in 2011, but the uP-SEP/Cr values in 2013 and 2016 decreased to 238.0 and 101.3. The eGFR also increased from 30, the indicator for GFR stage 5, and improved to stage 4. This case was later diagnosed as having drug-induced kidney injury (DKI), and a change in the drug was found to have improved the renal damage. These results indicate that the uP-SEP/Cr value is useful as a marker of tubulointerstitial disorders in CKD patients and can be used for patient monitoring. From the above results, it has become clear that cases with increased urinary presepsin concentrations are highly likely to have tubulointerstitial disorders. In other words, it has been found that tubulointerstitial disorders in CKD patients can be detected easily and early by using urinary presepsin as a marker.

According to the present invention, it is possible to detect and monitor tubulointerstitial disorders using the urinary presepsin concentration as an indicator. According to the present invention, in place of renal biopsy, it is possible to easily and early to grasp the degree of damage of tubulointerstitial disorders in patients for whom renal biopsy cannot be performed, and to grasp the degree of damage of tubulointerstitial disorders after treatment in patients for whom a treatment plan has been determined by renal biopsy, and it is possible to make a diagnosis. If it is possible to early grasp the degree of damage of tubulointerstitial disorders, active therapeutic intervention can be made at an early stage, leading to improved prognosis. The urinary presepsin concentration is also useful for estimating prognosis regarding renal function.

### INDUSTRIAL APPLICABILITY

According to the present invention, it is possible to detect the degree of renal damage in patients suspected of having tubulointerstitial disorders or patients with tubulointerstitial disorders by using the urinary presepsin value as an indicator. Furthermore, according to the present invention, it is possible to grasp the degree of renal damage in patients who cannot undergo renal biopsy or to grasp the degree of renal damage after treatment in patients whose treatment plan has been determined by renal biopsy, instead of renal biopsy, conveniently and at an early stage, and thus diagnosis becomes possible. Early grasp of the degree of renal damage will enable early and aggressive therapeutic intervention and improve the prognosis. Urinary presepsin value is also useful in grasping the severity of kidney disease.

Although the present invention has been described with reference to specific embodiments, various changes and modifications obvious to those skilled in the art are possible without departing from the scope of the appended claims.

## Claims

1. A method for detecting a tubulointerstitial disorder by measuring a concentration of sCD14-ST in urine of a subject (preferably a value obtained by dividing the concentration of sCD14-ST in urine by a urinary creatinine value).

2. The method according to claim 1, wherein the tubulointerstitial disorder is a chronic tubulointerstitial disorder.

3. The method according to claim 1 or 2, wherein the subject is a patient suspected of having a kidney disease or a patient with a kidney disease.

4. The method according to claim 1 or 2, wherein the subject is a patient suspected of having one or more kidney diseases selected from the group consisting of nephrosclerosis, IgA nephropathy, diabetic nephropathy, membranous nephropathy, ANCA-associated vasculitis, focal segmental glomerulosclerosis, minimal change nephrotic syndrome, lupus nephritis, tubulointerstitial nephritis, Sjogren's syndrome, vascular renal disease, idiopathic TINU syndrome, and IgG4-related nephritis, or a patient with said kidney diseases.

5. The method according to claim 1, wherein the subject is a patient suspected of having a kidney disease that is an acute tubulointerstitial nephritis or a chronic tubulointerstitial nephritis, or a patient with said kidney disease.

6. The method according to claim 1 or 5, wherein the subject is a patient suspected of having a kidney disease that is an acute drug-induced tubulointerstitial nephritis or a chronic drug-induced tubulointerstitial nephritis, or a patient with said kidney disease.

7. A method for monitoring a tubulointerstitial disorder by measuring a concentration of sCD14-ST in urine of a subject (preferably a value obtained by dividing the concentration of sCD14-ST in urine by a urinary creatinine value).

8. A method for selecting a treatment for a tubulointerstitial disorder by measuring a concentration of sCD14-ST in urine of a subject (preferably a value obtained by dividing the concentration of sCD14-ST in urine by a urinary creatinine value).

9. A method for grasping severity of a kidney disease by measuring a concentration of sCD14-ST in urine of a subject (preferably a value obtained by dividing the concentration of sCD14-ST in urine by a urinary creatinine value).

10. A method for detecting a kidney disease by measuring a concentration of sCD14-ST in urine of a subject (preferably a value obtained by dividing the concentration of sCD14-ST in urine by a urinary creatinine value).

11. A method for detecting a kidney disease by measuring a concentration of sCD14-ST in urine of a subject (preferably a value obtained by dividing the concentration of sCD14-ST in urine by a urinary creatinine value), wherein the kidney disease is one or more kidney diseases selected from the group consisting of nephrosclerosis, IgA nephropathy, diabetic nephropathy, membranous nephropathy, ANCA-associated vasculitis, focal segmental glomerulosclerosis, minimal change nephrotic syndrome, lupus nephritis, tubulointerstitial nephritis, Sjogren's syndrome, vascular renal disease, idiopathic TINU syndrome, and IgG4-related nephritis.

12. A method for detecting tubulointerstitial nephritis
by measuring a concentration of sCD14-ST in urine of a subject (preferably a value obtained by dividing the concentration of sCD14-ST in urine by a urinary creatinine value).

13. A method for detecting a tubulointerstitial disorder comprising:
measuring a concentration of sCD14-ST in urine collected from a patient suspected of having a tubulointerstitial disorder or a patient with a tubulointerstitial disorder (preferably a value obtained by dividing the concentration of sCD14-ST in urine by a urinary creatinine value), and
judging presence of a tubulointerstitial disorder when the concentration of sCD14-ST (preferably the value obtained by dividing the concentration of sCD14-ST in urine by a urinary creatinine value) is higher compared to healthy subjects.

14. A method for detecting and treating a tubulointerstitial disorder comprising:
measuring a concentration of sCD14-ST in urine collected from a patient suspected of having a tubulointerstitial disorder or a patient with a tubulointerstitial disorder (preferably a value obtained by dividing the concentration of sCD14-ST in urine by a urinary creatinine value),
judging presence of a tubulointerstitial disorder when the concentration of sCD14-ST (preferably the value obtained by dividing the concentration of sCD14-ST in urine by a urinary creatinine value) is higher compared to healthy subjects, and
performing immunosuppressive therapy when the presence of the tubulointerstitial disorder is judged.

15. A method for detecting a kidney disease comprising:
measuring a concentration of sCD14-ST in urine collected from a patient suspected of having a kidney disease or a patient with a kidney disease (preferably a value obtained by dividing the concentration of sCD14-ST in urine by a urinary creatinine value), and
judging presence of a kidney disease when the concentration of sCD14-ST (preferably the value obtained by dividing the concentration of sCD14-ST in urine by a urinary creatinine value) is higher compared to healthy subjects.

16. A method for detecting and treating a kidney disease comprising:
measuring a concentration of sCD14-ST in urine collected from a patient suspected of having a kidney disease or a patient with a kidney disease (preferably a value obtained by dividing the concentration of sCD14-ST in urine by a urinary creatinine value),
judging presence of a kidney disease when the concentration of sCD14-ST (preferably the value obtained by dividing the concentration of sCD14-ST in urine by a urinary creatinine value) is higher compared to healthy subjects, and
performing immunosuppressive therapy when the presence of the kidney disease is judged.

17. The method according to any one of claims 1 to 16, wherein the concentration of sCD14-ST (preferably the value obtained by dividing the concentration of sCD14-ST in urine by a urinary creatinine value) is measured by an immunoassay.

18. Use of a concentration of sCD14-ST in urine (preferably a value obtained by dividing the concentration of sCD14-ST in urine by a urinary creatinine value) as a marker for detecting a tubulointerstitial disorder.

19. A kit for detecting a tubulointerstitial disorder, comprising:
(a) an antibody specific for sCD14-ST,
(b) standard data correlating a concentration of sCD14-ST in urine (preferably a value obtained by dividing the concentration of sCD14-ST in urine by a urinary creatinine value) with a degree of renal damage, and
(c) an instruction manual.

20. A kit for detecting a tubulointerstitial disorder, comprising:
(a) an antibody specific for sCD14-ST,
(b) standard data correlating a concentration of sCD14-ST in urine (preferably a value obtained by dividing the concentration of sCD14-ST in urine by a urinary creatinine value) with severity of a kidney disease, and
(c) an instruction manual.


SEQUENCE LISTING

<110>  LSI Medience Corporation

<120>  Biomarker for detecting tubulointerstitial injury and its use

<130>  LSI-083

<150>  JP 2021-108341
<151>  2021-06-30

<160>  2

<170>  PatentIn version 3.5

<210>  1
<211>  11
<212>  PRT
<213>  Homo sapiens

<400>  1

Thr Thr Pro Glu Pro Cys Glu Leu Asp Asp Glu
1               5                   10

<210>  2
<211>  16
<212>  PRT
<213>  Homo sapiens

<400>  2

Arg Val Asp Ala Asp Ala Asp Pro Arg Gln Tyr Ala Asp Thr Val Lys
1               5                   10                  15